# EUROPEAN PATENT APPLICATION

(11) **EP 4 094 591 A1**
(43) Date of publication of application: **30.11.2022**
(21) Application number: 21176741.3
(22) Date of filing: 28.05.2021
(51) Int. Cl.: A23L 33/115, A23L 33/12, A23L 33/135, A23L 33/19, A23L 33/00, A61K 35/20, A61P 25/28

(54) **FOOD SUPPLEMENT**

(71) Applicant: Fonterra Co-Operative Group Limited, Auckland 1010 (NZ)
(72) Inventor: The designation of the inventor has not yet been filed
(74) Representative: Greaves Brewster LLP

(57) **Abstract**

This invention relates to the use of milk fat globule membrane (MFGM) or components thereof for improving developmental outcomes in an infant or child subject, in particular for improving social-emotional behaviour and improving memory.

## Description

### TECHNICAL FIELD

This invention relates to the use of milk fat globule membrane (MFGM) or components thereof for improving developmental outcomes in an infant or child subject, in particular for improving social-emotional behaviour and improving memory.

### BACKGROUND

The first 1000 days of life, from conception to the end of two years postpartum, is a critical phase during which the foundations of a child's development are laid. If a child's body and brain develop well, then their life chances are improved. Suboptimal nutrition during this period of life affects brain development, ranging from neuroanatomy, neurochemistry, neurophysiology, and neuropsychology to long-lasting influences on cognitive events well into adulthood⁽¹⁾. In the first 6 months, human milk is uniquely optimised for infant growth, neurodevelopment, and protection against infections and chronic diseases^{(2; 3)}. Breastfeeding is linked to higher intelligence in later life relative to formula feeding^{(4; 5)}, which may in part be due to compositional differences. The milk fat globule membrane (MFGM)^{(6; 7)} of mammalian milks comprises three polar lipid layers consisting of phosphatidylcholine, phosphatidylethanolamine, phosphatidylserine, sphingomyelin, plasmalogens, gangliosides, cholesterol, protein, and membrane-specific glycoproteins. Gangliosides are sialic-acid-containing glycosphingolipids in milk almost exclusively associated with the MFGM⁽⁸⁾. These complex lipids, gangliosides and phospholipids, are also found in high concentrations in the brain, where they influence membrane structural fluidity, synapse formation, neurogenesis, information storage, neurotransmission, and memory formation^{(9; 10; 11)}. Sphingolipids and cholesterol are essential components of myelin⁽¹²⁾, which is an electrical insulator and enhances cell signal transmission and cellular communication in the brain neural network⁽¹³⁾. These lipids also contribute to gut maturation and protection against respiratory and gastrointestinal infections in infancy and childhood^{(6; 14)}.

Human milk is the gold standard for feeding infants. However, when breastfeeding is not possible, infant and follow-on formulas are the only suitable alternative. Maternal formulas, infant formulas, follow-on formulas, growing-up formulas, dietetic products and other dairy containing compositions are typically produced using non-human milk. However, the nutritional composition of human milk differs in some respects to that of non-human milk. For example, non-human whole milk such as cow, goat or sheep milk, contains a higher proportion of saturated fatty acids than human milk and has lower levels of linoleic acid and alpha-linolenic acid, and polyunsaturated fatty acids that are essential for normal growth and development. Furthermore, milk fat is typically replaced with vegetable oils during the manufacture of pediatric formulas; the formulas are therefore depleted of the milk fat globule membrane (MFGM) and its components. Vegetable oils, which lack the bioactive lipid components of MFGM, have been used as the only source of lipids matching the fatty acid profile of human milk⁽⁷⁾. The recent commercial availability of bovine MFGM has increased its availability as a functional ingredient in infant formula⁽¹⁵⁾. Animal studies have shown that MFGM may alter brain lipid composition and functional and cognitive development, possibly through early up-regulation of the genes involved in brain function^{(6; 16; 17; 18)}. Clinical studies have shown that infants fed bovine MFGM supplemented formula, low in energy (60 in the treatment vs 66 kcal/100 mL in the control) and protein (1.20 in the treatment vs 1.27 g/100 mL in the control) from 2 to 6 months performed better in cognitive tests at 12 months using the Bayley Scales of Infant and Toddler Development III (Bayley-III) test, compared to infants fed standard formula in Swedish infants⁽¹⁹⁾. Milk lactoferrin with bovine MFGM resulted in a higher neurodevelopmental profile at 12 months and improved language subcategories at 18 months compared with a control group⁽²⁰⁾. Infant formula supplemented with phospholipids and gangliosides from the MFGM and with higher arachidonic acid improved cognitive development in healthy infants aged 0-6 months⁽²¹⁾. However, the responses in previous studies^{(19; 20; 21)} may not be due entirely to MFGM, as experimental and control formulae varied in more parameters than just their MFGM concentration.

It would be highly desirable to reduce any gaps in the cognitive development of formula-fed babies and children compared to breast-fed babies and children. It is therefore an object of the present invention to provide means for reducing any gaps in specific areas of cognitive development in formula-fed babies compared to breast-fed babies.

### SUMMARY OF THE INVENTION

A first aspect of the invention relates to the use of milk fat globule membrane (MFGM) and/or one or more of its components for improving social-emotional behaviour and/or improving memory in an infant or child subject.

A further aspect of the invention provides a method for improving social-emotional behaviour and/or improving memory in an infant or child subject, comprising administering a composition comprising MFGM and/or one or more of its components to a mother during gestation and/or postpartum during lactation, and/or to an infant or child subject in need thereof.

In another aspect the invention relates to a method of using MFGM and/or one or more of its components for improving social-emotional behaviour and/or improving memory, the method comprising providing a pregnant or lactating mother with a composition comprising MFGM and/or one or more of its components and optionally informing the mother that the composition will subsequently maintain or improve social-emotional behaviour and/or improve memory in her child in the weeks, months and years following birth.

Also provided by the invention is the use of MFGM and/or one or more of its components in the manufacture of a formulation for improving social-emotional behaviour and/or improving memory in an infant or child subject.

The invention also provides a food ingredient, food product or dietary supplement comprising, consisting of or essentially consisting of MFGM and/or one or more of its components for use in improving social-emotional behaviour and/or improving memory in an infant or child subject.

### DETAILED DESCRIPTION

A first aspect of the invention relates to the use of milk fat globule membrane (MFGM) and/or one or more of its components for improving social-emotional behaviour and improving memory in an infant or child subject.

The use comprises administering a composition comprising MFGM and/or one or more of its components to a mother during gestation and/or lactation, or to an infant or child subject in need thereof.

### Milk Fat Globule Membrane (MFGM)

The milk fat globule membrane (MFGM) is a well-known component of milk. It is composed primarily of lipids and proteins that surround milk fat globules secreted from the milk producing cells of humans and other mammals.

The MFGM has a phospholipid trilayer structure that contains other polar lipids (such as gangliosides (GA) and cerebrosides), MFGM proteins and cholesterol. The predominant MFGM phospholipids in human and bovine milk are phosphatidylcholine (PC), sphingomyelin (SM) and phosphatidylethanolamine (PE) with smaller amounts of phosphatidylserine (PS) and phosphatidylinositol (PI). Cholesterol and sphingomyelin form lipid rafts. The innermost layer of the MFGM is derived from the endoplasmic reticulum and the external bilayer of the MFGM derives from the apical plasma membrane of the mammary cell. Glycosylated lipids and proteins form the glycocalyx sticking out into the aqueous phase. This secretion process and the resulting membrane structure appear similar in all mammalian species' milk studied to date, (see Gallier et al., 2018, Agro Food Industry Hi Tech, Vol. 29(5).

With MFGM being a complex mixture of various proteins, lipids and other components, as described above, quantifying MFGM in its totality is currently not possible. The presence and amount of MFGM in a product is therefore determined by measuring one or more of its major components, such as phospholipids, gangliosides and MFGM proteins. Individual phospholipid species can be measured, for example, by HPLC-ELSD/CAD, ³¹P NMR, and HPLC-MS. Quantitative approaches for MFGM proteins include western blotting and HPLC-MS, and for glycosphingolipids such as GA, thin layer chromatography and HPLC-MS (see Gallier et al., 2018, Agro Food Industry Hi Tech, Vol. 29(5).

Reference herein to "components of MFGM", "MFGM components" and the like is taken to mean any one or more components that can be found in MFGM, such as one or more phospholipids, one or more sphingolipids, one or more sphingomyelins or derivatives thereof, one or more ceramides, one or more cerebrosides, one or more gangliosides, one or more MFGM proteins, or any combination thereof, which components are useful in improving social-emotional behaviour and improving memory in in an infant or child subject.

The one or more phospholipids may be selected from: phosphatidylcholine (PC), sphingomyelin (SM) and phosphatidylethanolamine (PE), phosphatidylserine (PS) and phosphatidylinositol (PI), or any combination thereof.

The one or more MFGM proteins may be selected from: Xanthine Oxidase, Butryophilin, Fatty acid binding protein, PAS6/7, adipophilin, Mucin 1, CD36, Mucin 15 and others, or any combination thereof.

The one or more gangliosides may be selected from: GM1, GM2, GM3, GM4, GD1, GD2, GD3, GT1, GT2, GT3, GQI, and GPI, and any one or more of the "a", "b", or "c" derivatives where they exist, and any combinations thereof.

Advantageously, administering MFGM to an infant or child subject, for example, through a suitable formulation, as described herein, raised serum ganglioside levels to levels similar to those seen in breast-fed subjects. In particular, MFGM-supplemented subjects showed increases in serum ganglioside levels or concentrations of GM3 (monosialodihexosylganglioside), GD3 (disialoganglioside 3), GM1 (monosialotetrahexosylganglioside), GDla (disialoganglioside 1a) and GDlb (disialoganglioside 1b), as well as in the sum of these gangliosides.

The technical effect of the present invention of improving social-emotional behaviour and improving memory in an infant or child subject may also therefore advantageously be achieved through the administration to a subject of components of MFGM, particularly gangliosides, such as any one or more of GM1: monosialotetrahexosylganglioside; GM2: monosialoganglioside 2; GM3: monosialodihexosylganglioside; GD1a: disialoganglioside 1a; GD1b: disialoganglioside 1b; GD3: disialoganglioside 3. According to a preferred embodiment, the ganglioside is any one or more of: GM3, GD3, GM1, GDla and GDlb or similar, and any combinations thereof.

The ganglioside(s) may be dietary ganglioside(s).

Gangliosides are a group of sialic-acid-containing glycosphingolipids and may be found in animal tissues and fluids, such as blood, brain tissue and milk. Gangliosides may be extracted from milk or from other animal sources, such as beef, chicken, pork, and fish and marine species, such as sea urchin. Gangliosides may also be produced synthetically or semi-synthetically.

Gangliosides may be measured using known techniques, such as by measuring Lipid Bound Sialic Acid (LBSA) or individual species quantified by Thin Layer Chromatography (TLC), High Performance Liquid Chromatography with UV detection (HPLC-UV), or by liquid chromatography linked to mass spectrometry. A number of techniques can be employed to extract gangliosides from a variety of materials according to methods well known to those skilled in the art.

MFGM or components thereof may suitably be derived from any mammalian milk, such as cow, buffalo, goat, sheep or human milk.

MFGM or components thereof may be extracted from milk or other dairy sources, particularly any high fat milk fraction, for example: cream, butter, buttermilk, butter serum, beta serum and whey protein concentrates enriched in MFGM. Advances in dairy processing have made possible the MFGM enrichment of dairy streams, which has given the ability to match the human milk fat globule composition and structure. Preferably, MFGM or components thereof are produced through enrichment of dairy streams to produce food products or ingredients enriched in MFGM or components thereof.

Milk fat globule membrane material may be isolated according to the acidification method of Kanno & Dong-Hyun, 1990, and further fractionated into lipid and protein fractions by the addition of methanol, as described by Kanno *et al.,* 1975. A phospholipid extract may be isolated by extracting the lipid mixture with acetone according to the procedure of Purthi *et al.,* 1970. Lipid residue may be further enriched in milk fat globule membrane lipids by the selective extraction of non-polar lipids with pentane.

MFGM and MFGM components are also commercially available under, for example, the product names SureStart^{™} and Lacprodan^{™}.

### Social-Emotional Behaviour

An improvement in "social-emotional" behaviour may be measured using the Bayley Scales of Infant and Toddler Development, Third Edition ("Bayley-III"), see Chapter 5 "The Bayley-III Social-Emotional Scale". An improvement in social-emotional behaviour may be manifested as an improvement in the score at any one of more of stages 1, 2, 3, 4a, 4b, 5a, 5b or 6, as shown in the table below.

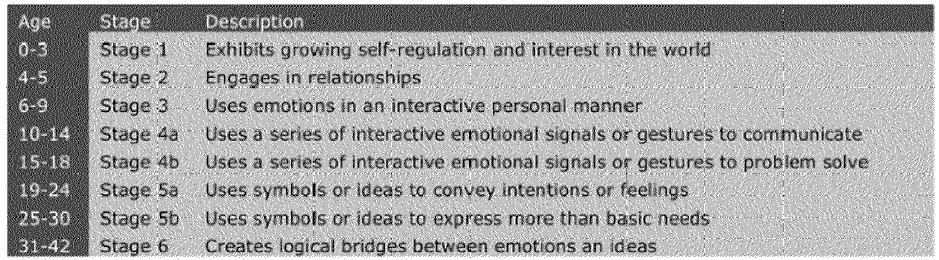

A person skilled in the art would readily be able to conduct an evaluation on a suitable subject using the Bayley-III scale and to obtain a score for the subject and determine whether the subject falls within the normal range for their age.

For children above the age of 42 months, other tests are available, which would be well known to those skilled in the art. Examples include IRC Social and Emotional Learning Competencies; Four branch model of Emotional Intelligence assessments; International social and emotional learning assessment (ISELA); and Behavioural Assessment of the Dysexecutive Syndrome in Children. See Isaacs and Oates (Eur J Nutr (2008) 47{Suppl 3}:4-24) and Thomson et al., 2018 (Journal of Applied Developmental Psychology 55, pp. 107-118) for further details of known tests.

An improvement in social-emotional behaviour may be relative to non-MFGM supplemented subjects. An improvement may also be registered when a smaller difference is seen between the scores of an MFGM-supplemented subject and breast-fed subjects compared to the difference between the scores of non-MFGM supplemented subjects and breast-fed subjects. Reference herein to breast-fed subjects refers to infants who are breast-fed at least 90% of the time for the first four to six months of life. Reference herein to non-breast-fed subjects refers to subjects who receive formula-milk, whether supplemented or not, at least 90% of the time for the first twelve months of life.

### Memory

Various methods for the assessment of memory are known in the art. Examples include Bayley Scales of Infant and Toddler Development, Third Edition ("Bayley-III"), see Chapter 2 "The Bayley-III Cognitive Scale". Other known memory tests include Children's Memory Scale (CMS), 1997, Morris Cohen. Further tests are described in Isaacs and Oates (Eur J Nutr (2008) 47{Suppl 3}:4-24), see page 14-15 in particular.

The improvement in memory is preferably an improvement in short-term memory. The assessment of memory, including short-term memory, and what constitutes an improvement will vary depending on the developmental stage / age of the subject.

An improvement in memory, including short-term memory, may be relative to non-MFGM supplemented subjects of the same age. An improvement may also be registered when a smaller difference is seen between the scores or results of an MFGM-supplemented subject and breast-fed subjects compared to the difference between the scores or results of non-MFGM supplemented subjects and breast-fed subjects of the same age.

Reference herein to breast-fed subjects refers to infants who are breast-fed at least 90% of the time for the first four to six months of life. Reference herein to non-breast-fed subjects, i.e. to those receiving formula, whether or not supplemented with MFGM, refers to subjects who receive formula-milk at least 90% of the time for the first twelve months of life.

### Food Product, Ingredient, Dietary Supplement

The MFGM and/or components thereof may be comprised in a food product or may be used as an ingredient or provided as a dietary supplement. The MFGM and/or components thereof may be also comprised in a formulation. MFGM and/or components thereof may be provided in the form of MFGM-enriched products and ingredients.

MFGM or components thereof may be formulated as a food, drink, food additive or ingredient, drink additive or ingredient, dietary supplement, nutritional product, medical food, enteral or parenteral feeding product, meal replacement, or pharmaceutical.

The food product may be an infant, follow-on, growing up, or maternal formula. The term "infant formula" as referred to herein is taken to mean a composition for infants aged between 0 days and 6 months old and a "follow-on formula" refers to a composition for infants aged 6 months to 1 year. The term "growing up formula" as used herein refers to compositions directed to infants and children aged 1 year upwards. Growing-up formula includes growing-up milk powders or GUMPs. It will be appreciated by those skilled in the art that the age ranges for the different compositions: "infant formula", "follow-on formula" and "growing-up formula" can vary from child to child depending on the individual's development. The term "maternal formula" as used herein refers to a composition to be taken by pregnant or lactating women. The food product can also be a dietetic product, which refers to a product specially processed or formulated to satisfy particular dietary requirements which exist because of a particular physical or physiological condition and/or specific diseases and disorders and which are presented as such. The aforementioned products may be in liquid form as concentrates or ready-to-drink liquids or provided as powder concentrates.

The MFGM-containing food product may also be any other dairy product, such as yoghurt, cheese, cream, buttermilk, beta serum, butter serum, high fat whey protein concentrate, high fat milk protein concentrate, high fat whey protein isolate, high fat milk protein isolate or fractions thereof, or MFGM or one or more components thereof may be derived from any of the aforementioned products. The products may themselves be ingredients for further use in other areas of the food and drinks industry. The products may be MFGM-enriched products and ingredients, produced through the enrichment of dairy streams.

"Beta-serum" refers to an aqueous dairy ingredient separated from dairy streams containing greater than 60% fat that have been through phase inversion from an oil-in-water to a water-in-oil emulsion, as described below. Cream is the preferred starting material for the production of beta-serum. For example, beta-serum is produced during the production of butter-oil (also known as anhydrous milk fat or AMF) from cream. Preferably the beta serum is dried; preferably dried beta-serum is a powder.

The MFGM-containing food product may also be a non-dairy product such as baby food, fruit juices, (baby) rice, rusks, puree, or porridge.

The MFGM and/or components thereof may be provided as a dietary supplement, such as in the form of oil drops, gummy, tablet, a caplet, a pill, a hard or soft capsule, or lozenge. The supplement may be in the form of a sachet, dispensable powder, granules, suspension, an elixir, a liquid, or any other form that can be added to food or drink, including for example water, milk or fruit juice.

The food products, ingredients or dietary supplements may further comprise probiotics and/or prebiotics. Suitable prebiotics may include human milk oligosaccharides, galacto-oligosaccharides, fructo-oligosaccharides and inulin. Suitable prebiotics may include bifidobacteria, lactobacilli, *Bifidobacterium animalis* subsp. *lactis* HN019 (HN019), and *Lactobacillus rhamnosus* HN001 (HN001).

The food products, ingredients or dietary supplements may further comprise nutrients to aid learning, behaviour, social and emotional development. Such nutrients may include polyunsaturated fatty acids (such as docosahexaenoic acid (DHA), omega 3, omega 6), prebiotics and probiotics, which products may be acting through the gut brain axis.

A second aspect of the invention provides a method for improving social-emotional behaviour and/or improving memory in an infant or child subject, comprising administering a formulation comprising MFGM and/or one or more of its components to a mother during gestation or lactation or to an infant or child subject in need thereof.

The formulation is preferably administered orally. The term "oral administration" includes oral, buccal, enteral and intra-gastric administration.

The formulation may comprise a suitable carrier, diluent or excipient, or combination thereof, to allow the formulation to be effectively administered to a subject without reducing the activity of the MFGM and/or one or more of its components.

The "subject" of the invention is a foetus, infant or child. The term "infant" or "child" as defined herein is taken to mean a child of any age from birth onwards, including premature babies, until pre-pubescence, anytime between the ages of ten and fourteen. Where the subject is a foetus, the formulation is administered to the expectant mother. The expectant mother may suitably be up to one, two, three, four, five, six, seven, eight or nine months pregnant. The formulation of the invention may also be administered postpartum to a lactating mother, whereby the formulation is delivered to the baby or infant through breastfeeding and/or through supplementation of expressed milk. Where the formulation is administered to the expectant mother or postpartum, the resulting improvement is seen in the infant or child in the weeks, months and years after the birth. The improvement in social-emotional behaviour and in memory may be seen from birth onwards until the child is up to one month, two months, three months, four months, five months, six months, seven months, eight months, nine months, ten months, eleven months or one year, two years, three years, four years, five years, six years, seven years, eight years, nine years, ten years, eleven years, or up to twelve years of age, and suitably where the child is not fed an MFGM-supplemented formula nor substantially breast-fed.

A further aspect of the invention relates to a method of using MFGM and/or one or more of its components for improving social-emotional behaviour and/or improving memory, the method comprising providing a pregnant mother with a formulation comprising MFGM and/or one or more of its components and optionally informing the mother that the formulation will subsequently maintain or improve social-emotional behaviour and/or increase memory in her child once born. The improvement in social-emotional behaviour and/or in memory may be seen from birth onwards until the child is up to one month, two months, three months, four months, five months, six months, seven months, eight months, nine months, ten months, eleven months or one year, two years, three years, four years, five years, six years, seven years, eight years, nine years, ten years, eleven years, or up to twelve years of age, and suitably where the child is not fed an MFGM-supplemented formula nor substantially breast-fed.

An effective amount of the formulation is administered to the mother during gestation and/or lactation, or to the infant or child subject, an effective amount being the amount required to confer an improvement in social-emotional behaviour in an infant or child subject. The interrelationship of dosages for animals and humans (based on milligrams per meter squared of body surface) is described by Freireich, et al. (1966). Body surface area can be approximately determined from height and weight of the subject. See, e.g., Scientific Tables, Geigy Pharmaceuticals, Ardley, New York, 1970, 537. Effective doses also vary, as recognised by those skilled in the art, dependent on route of administration, carrier usage, and the like.

Formulations suitable for use in the invention may comprises at least about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9% or at least about 1% gangliosides w/w on a dry weight basis. Alternatively, formulations useful in the invention may comprises at least about 1, 2, 3, 4, 5, 6, 7, 8, 9, 10, 11, 12, 13, 14, 15, 16, 17, 18, 19, 20, 30, 40, 50, 60, 70, 80, 90, 100, 150, 200, 250, 300, 350, 400, 450, 500, 550, 600, 650, 700, 750, 800, 850, 900, 950, 1000, 2000, 3000, 4000, 5000, 6000, 7000, 8000, 9000, or at least about 10000 mg or more gangliosides per 100g dry weight.

Alternatively the formulation when administered during pregnancy or lactation is formulated to provide at least about 1, 2, 3, 4, 5, 6, 7, 8, 9, 10, 11, 12, 13, 14, 15, 16, 17, 18, 19 or 20, 30, 40, 50, 60, 70, 80, 90, or at least about 100 mg, preferably at least about 10-50 mg, preferably at least about 7.5 mg gangliosides per day to the mother, and useful ranges may be chosen between any of these values (for example, about 1 to about 100 mg gangliosides per day to the mother, or about 2 to about 60 mg gangliosides per day to the mother, or about 5 to about 40 mg gangliosides per day to the mother, or 5 to about 30 mg gangliosides per day to the mother).

For example, a formulation (infant, follow-on, growing up or maternal) useful herein may comprise:
(a) 80-99.9% of a milk powder selected from whole milk powder, skim milk powder, milk protein concentrate (MPC), milk protein isolate (MPI); and whey protein such as a WPC or WPI;
(b) 0- 20% lipid such as milk fat or one or more vegetable oil
(c) 0-25% sugars or carbohydrate ingredient
(d) 0.1-0.5% vitamin and mineral mix
(e) 0-5% flavour ingredients, and
(f) 1-20% MFGM or one or more components thereof.

Part (f) above may also be 1-50% MFGM or one or more components thereof, with parts (a) to (e) adjusted accordingly.

For example, when administered in an infant formula or follow-on formula, the MFGM may comprise, consist of, or essentially consist of each of the following components per 100g, or of any one or more of components (a) to (g) per 100g:

| | |
|---|---|
| (a) Phospholipids: | 400-700 mg |
| (b) Phosphatidylcholine: | 100-200 mg |
| (c) Phosphatidylethanolamine: | 100-200 mg |
| (d) Phosphatidylinositol: | 30-65 mg |
| (e) Phosphatidylserine: | 35-95 mg |
| (f) Sphingomyelin: | 90-200 mg |
| (g) Gangliosides: | 18-65 mg |

When administered in a growing up milk, the MFGM may comprise, consist of or essentially consist of each of the following components per 100g, or of any one or more of components (a) to (g) per 100g:

| | |
|---|---|
| (a) Phospholipids: | 300-600 mg |
| (b) Phosphatidylcholine: | 75-160 mg |
| (c) Phosphatidylethanolamine: | 70-160 mg |
| (d) Phosphatidylinositol: | 20-50 mg |
| (e) Phosphatidylserine: | 30-70 mg |
| (f) Sphingomyelin: | 70-150 mg |
| (g) Gangliosides: | 5-20 mg |

When administered in a maternal milk, the MFGM may comprise, consist of or essentially consist of each of the following components per 100g, or of any one or more of components (a) to (g) per 100g:

| | |
|---|---|
| (a) Phospholipids: | 300-600 mg |
| (b) Phosphatidylcholine: | 75-160 mg |
| (c) Phosphatidylethanolamine: | 70-160 mg |
| (d) Phosphatidylinositol: | 20-50 mg |
| (e) Phosphatidylserine: | 30-70 mg |
| (f) Sphingomyelin: | 70-150 mg |
| (g) Gangliosides: | 5-20 mg |

The maternal milk may, for example, be provided in two daily servings of 37.5g to give a total of 75g/day, delivering the following amounts per day. The respective quantities of elements (a) to (g) would of course vary for a smaller or greater serving size or according to the number of servings.

| | |
|---|---|
| (a) Phospholipids: | 225-450 mg |
| (b) Phosphatidylcholine: | 55-120 mg |
| (c) Phosphatidylethanolamine: | 50-120 mg |
| (d) Phosphatidylinositol: | 15-40 mg |
| (e) Phosphatidylserine: | 20-40 mg |
| (f) Sphingomyelin: | 50-115 mg |
| (g) Gangliosides: | 4-15 mg |

The growing up milk may, for example, be provided in two daily servings of 28g to give a daily total of 56g/day, or in three daily servings of 28g to give a daily total of 84g/day delivering the following amounts. The respective quantities of elements (a) to (g) would of course vary for a smaller or greater serving size or according to the number of servings.

| | |
|---|---|
| (a) Phospholipids: | 165-500 mg |
| (b) Phosphatidylcholine: | 40-135 mg |
| (c) Phosphatidylethanolamine: | 40-135 mg |
| (d) Phosphatidylinositol: | 10-40 mg |
| (e) Phosphatidylserine: | 15-60 mg |
| (f) Sphingomyelin: | 40-126 mg |
| (g) Gangliosides: | 3-17 mg |

The feeding requirements of babies will vary greatly as they grow. The table below shows the typical recommended feeds per day according to age and the respective quantities of MFGM components.

| Age of infant (months) | Recommended feeds/day | Number of scoops (4.4g) | Amount of powder consumed each day | Volume of formula per day (ml) | **Phospholipid** | **PC** | **PE** | **SM** | **PI** | **PS** | **GA** |
|---|---|---|---|---|---|---|---|---|---|---|---|
| 0-2 weeks | 7 | 2 | 61.6 | 467 | **245-500** | **60-140** | **60-140** | **55-140** | **18-50** | **20-70** | **9-50** |
| 0-2 weeks | 8 | 2 | 70.4 | 533 | | | | | | | |
| 2-4 weeks | 7 | 3 | 92.4 | 700 | **370-740** | **90-210** | **90-210** | **80-210** | **25-70** | **30-100** | **16-68** |
| 2-4 weeks | 8 | 3 | 105.6 | 800 | | | | | | | |
| 1-2 months | 6 | 4 | 105.6 | 800 | **420-860** | **105-250** | **105-250** | **95-250** | **30-80** | **35-120** | **19-80** |
| 1-2 months | 7 | 4 | 123.2 | 933 | | | | | | | |
| 2-4 months | 5 | 5 | 110 | 833 | **440-925** | **110-265** | **110-265** | **100-265** | **33-85** | **38-125** | **20-86** |
| 2-4 months | 6 | 5 | 132 | 1000 | | | | | | | |
| 4-6 months | 5 | 6 | 132 | 1000 | **528-1105** | **130-320** | **130-320** | **120-320** | **40-105** | **45-150** | **23-105** |
| 4-6 months | 6 | 6 | 158.4 | 1200 | | | | | | | |

These formulations may also comprise 0.1 to 4% w/w, preferably 2 to 4% w/w, of one or more of a vitamin premix, a mineral premix, lecithin, one or more antioxidants, one or more stabilisers, or one or more nucleotides, or a combination of any two or more thereof. The formulations may also comprise flavorings.

In some embodiments, these formulations may be formulated to provide from about 2700 to about 3000 kJ/L.

The formulation may be a liquid (concentrate or ready-to-drink) or powdered maternal formula, infant formula, follow-on formula, growing-up formula or dietetic product.

The formulation may comprise at least about 0.1, 0.2, 0.3, 0.4, 0.5, 0.6, 0.7, 0.8, 0.9, 1.0, 1 5, 2, 2.5, 3, 3.5, 4, 4.5, 5, 5.5, 6, 6.5, 7, 7.5 8, 8.5, 9, 9.5, 10, 15, 20, 25, 30, 35, 40, 45, 50, 55, 60, 65, 70, 75, 80, 85, 90, 95, 99 or 99.5% by weight total lipid, and useful ranges may be selected between any of these values (for example, about 5 to about 95%, about 10 to about 95%, about 15 to about 95%, about 20 to about 95%, about 25 to about 95%, about 30 to about 95%, about 35 to about 95%, about 40 to about 95%, about 45 to about 95%, about 50 to about 95%, about 5 to about 99%, about 10 to about 99%, about 15 to about 99%, about 20 to about 99%, about 25 to about 99%, about 30 to about 99%, about 35 to about 99%, about 40 to about 99%, about 45 to about 99%, about 50 to about 99%, about 5 to about 70%, about 10 to about 70%, about 15 to about 70%, about 20 to about 70%, about 25 to about 70%, about 30 to about 70%, about 35 to about 70%, about 40 to about 70%, about 45 to about 70%, and about 50 to about 70% by weight total lipid).

Additionally or alternatively, the formulation may comprise at least about 0.1, 0.2, 0.3, 0.4, 0.5, 0.6, 0.7, 0.8, 0.9, 1.0, 1 5, 2, 2.5, 3, 3.5, 4, 4.5, 5, 5.5, 6, 6.5, 7, 7.5 8, 8.5, 9, 9.5, 10, 15, 20, 25, 30, 35, 40, 45, 50, 55, 60, 65, 70, 75, 80, 85, 90, 95, 99 or 99.5% by weight phospholipid, and useful ranges may be selected between any of these values (for example, about 5 to about 95%, about 10 to about 95%, about 15 to about 95%, about 20 to about 95%, about 25 to about 95%, about 30 to about 95%, about 35 to about 95%, about 40 to about 95%, about 45 to about 95%, about 50 to about 95%, about 5 to about 99%, about 10 to about 99%, about 15 to about 99%, about 20 to about 99%, about 25 to about 99%, about 30 to about 99%, about 35 to about 99%, about 40 to about 99%, about 45 to about 99%, about 50 to about 99%, about 5 to about 70%, about 10 to about 70%, about 15 to about 70%, about 20 to about 70%, about 25 to about 70%, about 30 to about 70%, about 35 to about 70%, about 40 to about 70%, about 45 to about 70%, and about 50 to about 70% by weight phospholipid).

Additionally or alternatively, the formulation may comprise at least about 0.1, 0.2, 0.3, 0.4, 0.5, 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30% by weight of one or more phospholipids selected independently from phosphatidylcholine, phosphatidylethanolamine, sphingomyelin, phosphatidylserine, and phosphatidylinositol, and useful ranges may be selected between any of these values (for example, about 0.1 to about 30%, about 0.5 to about 30%, about 1 to about 30%, about 2 to about 30%, about 3 to about 30%, about 4 to about 30%, about 5 to about 30%, about 10 to about 30%, about 15 to about 30%, about 20 to about 30%, about 0.1 to about 5%, about 0.5 to about 5%, about 1 to about 5%, about 2 to about 5%, about 3 to about 5%, about 0.1 to about 10%, about 0.5 to about 10%, about 1 to about 10%, about 2 to about 10%, about 3 to about 10%, about 4 to about 10%, about 5 to about 10%, about 6 to about 10%, about 0.1 to about 20%, about 0.5 to about 20%, about 1 to about 20%, about 2 to about 20%, about 3 to about 20%, about 4 to about 20%, about 5 to about 20%, about 10 to about 20%, about 15 to about 20% by weight of one or more phospholipids selected independently from phosphatidylcholine, phosphatidylethanolamine, sphingomyelin, phosphatidylserine, and phosphatidylinositol).

Additionally or alternatively, the formulation may comprise at least about 0.1, 0.2, 0.3, 0.4, 0.5, 0.6, 0.7, 0.8, 0.9, 1.0, 1 5, 2, 2.5, 3, 3.5, 4, 4.5, 5, 5.5, 6, 6.5, 7, 7.5 8, 8.5, 9, 9.5, 10, 15, 20, 25, 30, 35, 40, 45, 50, 55, 60, 65, 70, 75, 80, 85, 90, 95 or 99.5% by weight ganglioside, and useful ranges may be selected between any of these values (for example, about 5 to about 95%, about 10 to about 95%, about 15 to about 95%, about 20 to about 95%, about 25 to about 95%, about 30 to about 95%, about 35 to about 95%, about 40 to about 95%, about 45 to about 95%, about 50 to about 95%, about 10 to about 70%, about 15 to about 70%, about 20 to about 70%, about 25 to about 70%, about 30 to about 70%, about 35 to about 70%, about 40 to about 70%, about 45 to about 70%, and about 50 to about 70% by weight phospholipid). The formulation may comprise one or more gangliosides selected from GM1, GM2, GM3, GM4, GDI, GD2, GD3, GT1, GT2, GT3, GQI, and GPI, and any one or more of the "a", "b", or "c" derivatives where they exist, and any combination thereof. Preferably, the gangliosides in the formulation comprise, consist of or essentially consist of the following: GM3, GD3, GM1, GD1a, GD1b, or any combination thereof.

Additionally or alternatively, the formulation may comprise at least about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 3, 4, 5, 6, 7, 8, 9, 10, 11, 12, 13, 14, 15, 16, 17, 18, 19, 20, 21, 22, 23, 24, 25, 26, 27, 28, 29 or 30% by weight of one or more gangliosides, optionally selected independently from GM3, GD3, GM1, GDla and GD1b, and useful ranges may be selected between any of these values (for example, about 0.1 to about 30%, about 0.5 to about 30%, about 1 to about 30%, about 2 to about 30%, about 3 to about 30%, about 4 to about 30%, about 5 to about 30%, about 10 to about 30%, about 15 to about 30%, about 20 to about 30%, about 0.1 to about 5%, about 0.5 to about 5%, about 1 to about 5%, about 2 to about 5%, about 3 to about 5%, about 0.1 to about 10%, about 0.5 to about 10%, about 1 to about 10%, about 2 to about 10%, about 3 to about 10%, about 4 to about 10%, about 5 to about 10%, about 6 to about 10%, about 0.1 to about 20%, about 0.5 to about 20%, about 1 to about 20%, about 2 to about 20%, about 3 to about 20%, about 4 to about 20%, about 5 to about 20%, about 10 to about 20%, about 15 to about 20% by weight of one or more gangliosides selected independently from GM3, GD3, GM1, GDla and GD1b.

The formulation may comprise about 15% to about 99% by weight total lipid, about 1% to about 80% by weight phospholipid, about 1% to about 25% by weight phosphatidylcholine, about 0.1% to about 15% by weight phosphatidylinositol, about 0.1% to about 15% by weight phosphatidylserine, about 1% to about 30% by weight phosphatidylethanolamine, about 0.5% to about 25% by weight sphingomyelin, and about 0.1 to about 10% by weight ganglioside. In some embodiments the formulation comprises one or more gangliosides comprises about 1% to about 60% by weight lactose, about 1% to about 15 % by weight lactose or about 50% to about 65% by weight lactose.

A third aspect of the invention provides the use of MFGM and/or one or more of its components in the manufacture of a formulation for improving social-emotional behaviour and/or improving memory in an infant or child subject.

The formulation may comprise, consist essentially of, or consist of about 0.1, 0.5, 1, 5, 10, 15, 20, 25, 30, 35, 40, 45, 50, 55, 60, 65, 70, 75, 80, 85, 90, 95, 99, or 100% by weight of fresh, recombined or powdered whole milk or a milk derivative and useful ranges may be selected between any of these foregoing values (for example, from about 0.1 to about 50%, from about 0.2 to about 50%, from about 0.5 to about 50%, from about 1 to about 50%, from about 5 to about 50%, from about 10 to about 50%, from about 15 to about 50%, from about 20 to about 50%, from about 25 to about 50%, from about 30 to about 50%, from about 35 to about 50%), from about 40 to about 50%, and from about 45 to about 50%). The milk derivative is preferably selected from recombined, powdered or fresh skimmed milk, reconstituted whole or skimmed milk powder, skimmed milk concentrate, skim milk retentate, concentrated milk, ultrafiltered milk retentate, milk protein concentrate (MPC), milk protein isolate (MPI), calcium depleted milk protein concentrate (MPC), low fat milk, low fat milk protein concentrate (MPC), casein, caseinate, milk fat, cream, butter, ghee, anhydrous milk fat (AMF), buttermilk, butter serum, beta serum, hard milk fat fractions, soft milk fat fractions, sphingolipid fractions, milk fat globule membrane fractions, phospholipid fractions, complex lipid fractions, colostrum, a colostrum fraction, colostrum protein concentrate (CPC), colostrum whey, an immunoglobulin fraction from colostrum, whey, whey protein isolate (WPI), whey protein concentrate (WPC), sweet whey, lactic acid whey, mineral acid whey, reconstituted whey powder, a composition derived from any milk or colostrum processing stream, a composition derived from the retentate or permeate obtained by ultrafiltration or micro filtration of any milk or colostrum processing stream, a composition derived from the breakthrough or adsorbed fraction obtained by chromatographic (including but not limited to ion and gel permeation chromatography) separation of any milk or colostrum processing stream, extracts of any of these milk derivatives including extracts prepared by multistage fractionation, differential crystallisation, solvent fractionation, compressed gas extraction (such as supercritical fractionation, near supercritical fractionation), distillation, centrifugal fractionation, or fractionation with a modifier (e.g. soaps or emulsifiers), hydrolysates of any of these derivatives, fractions of the hydrolysates, and combinations of these derivatives, including combinations of hydrolysed and/or non-hydrolysed fractions. It should be understood that the source of these derivatives may be milk or colostrum or a combination thereof. It should also be understood that the milk fat may be provided as fresh, recombined or powdered whole milk, one or more milk derivatives as described above, or combinations thereof.

The formulation may further comprise a suitable carrier. The formulation may be formulated as a food, drink, food additive, drink additive, dietary supplement, nutritional product, medical food, enteral or parenteral feeding product, meal replacement, nutraceutical, medicament or pharmaceutical. The formulation may be in the form of oil drops, a tablet, a caplet, a pill, a hard or soft capsule, a gummy or a lozenge. The formulation may be in the form of a sachet, a dispensable powder, granules, a suspension, an elixir, a liquid, or any other form that can be added to food or drink, including for example water, milk or fruit juice. The formulation may further comprise one or more constituents (such as antioxidants) which prevent or reduce degradation of the formulation during storage or after administration. These formulations may include any edible consumer product which is able to carry lipids. Examples of suitable edible consumer products include aqueous products, baked goods, confectionary products including chocolate, gels, ice creams, reconstituted fruit products, snack bars, food bars, muesli bars, spreads, sauces, dips, dairy products including yoghurts and cheeses, drinks including dairy and non-dairy based drinks, milk, milk powders, sports supplements including dairy and non-dairy based sports supplements, fruit juice, food additives such as protein sprinkles and dietary supplement products including daily supplement tablets. Suitable nutraceutical compositions useful herein may be provided in similar forms.

A fourth aspect of the invention provides a food ingredient, food product or dietary supplement comprising, consisting of or essentially consisting of MFGM and/or one or more of its components, or a formulation as described herein, for use in improving social-emotional behaviour and/or improving memory in an infant or child subject.

A formulation useful herein may be formulated as a food, drink, food additive, drink additive, dietary supplement, nutritional product, medical food, enteral or parenteral feeding product or meal replacement. Appropriate formulations may be prepared by an art skilled worker with regard to that skill and the teaching of this specification.

As will be appreciated, the dose of the formulation administered, the period of administration, and the general administration regime may differ between subjects depending on such variables as the mode of administration chosen, and the age, sex and/or general health of a subject.

In one embodiment, formulations useful herein include maternal formulas, infant formulas, follow-on formulas and growing up formulas, in liquid (concentrate or ready-to-drink) or powder form. Such products are formulated to target nutrients to the foetus, infant and child. It is appreciated by the first life-stages (foetus, infant and growing child) involve significant growth and development. Any support which enhances development can have significant effects on the development of the individual.

In another embodiment, formulations useful herein include dietetic products.

In alternative embodiments, the formulations useful herein may be formulated to allow for administration to a subject by any chosen route, including but not limited to oral administration.

Throughout the description and claims of this specification, the words "comprise" and "contain" and variations of the words, for example "comprising" and "comprises", mean "including but not limited to", and do not exclude other components, integers or steps.

Moreover, the singular encompasses the plural unless the context otherwise requires: in particular, where the indefinite article is used, the specification is to be understood as contemplating plurality as well as singularity, unless the context requires otherwise.

Preferred features of each aspect of the invention may be as described in connection with any of the other aspects. Within the scope of this application, it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and die highest value enumerated are to be considered to be expressly stated in this application in a similar manner.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

### BRIEF DESCRIPTION OF THE DRAWINGS

One or more embodiments of the invention will now be described, by way of example only, with reference to the accompanying drawings, in which:

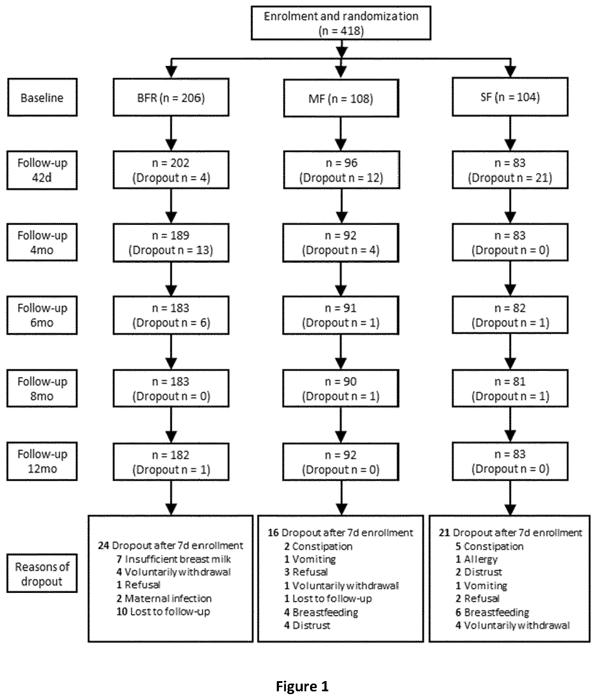

**Figure 1** shows a flow chart of change in infant numbers in the MF (MFGM-enriched formula), SF (standard formula), and BFR (breast-fed reference) groups during the study period and reasons for dropping out of the study.

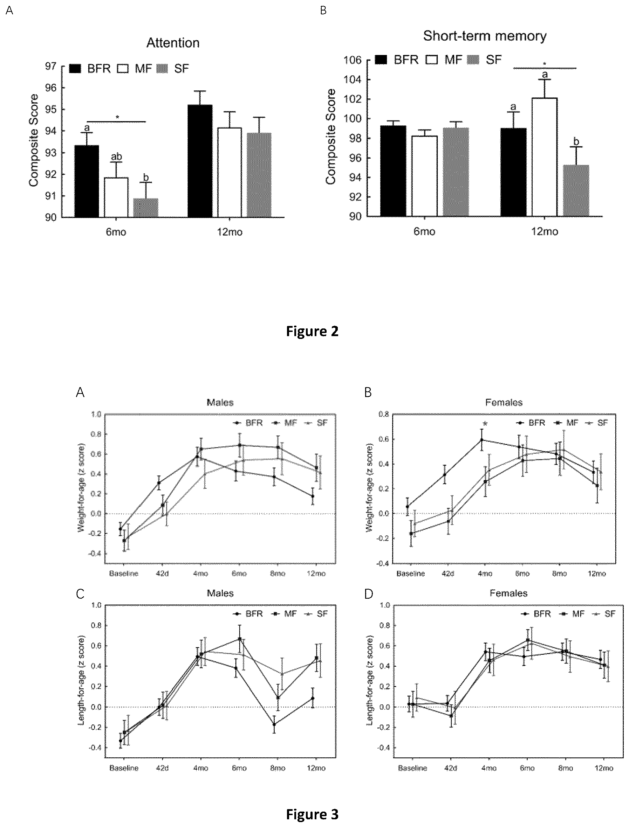

**Figure 2** shows the composite attention and short-term memory scores, presented as mean ± SE, in the MF group (white column), SF group (grey column), and BFR group (black column) at 6 and 12 months of age. **A.** Composite attention score among the three groups. **B.** Composite short-term memory score among the three groups: MF, SF and BFR. Data were analysed using a general linear model with univariate analysis adjusted for socioeconomic factors (maternal age, parental education, and family income) with group and sex as fixed effects, group by sex interaction, group and time interaction and site as a random factor.

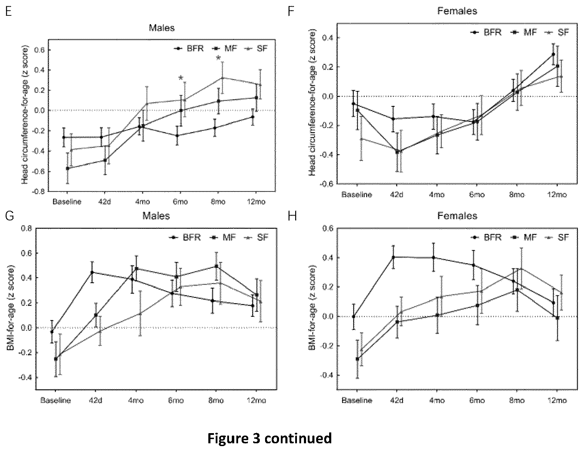

**Figure 3** shows the dynamic variation of Z-scores (mean ± SD, 95% CI) of infants from 0 to 12 months in MF (blue line, n=108), SF (red line, n=104), and BFR (black line, n=206) groups. Weight-for-age Z-score in males (A) and females (B); length-for-age Z-score in males (C) and females (D); head-circumference-for-age Z-score in males (E) and females (F); BMI (body mass index)-for-age Z-score in males (G) and female (H). Data were analysed using a general linear mixed model repeated measures with six time points adjusted for group and sex as fixed effects, group by sex interaction and site as a random effect with post hoc Bonferroni adjusted comparisons when the overall comparison was significant. To investigate potential different time trends within each group, the interaction between time and group were included in the model. *P< 0.05 (overall comparison).

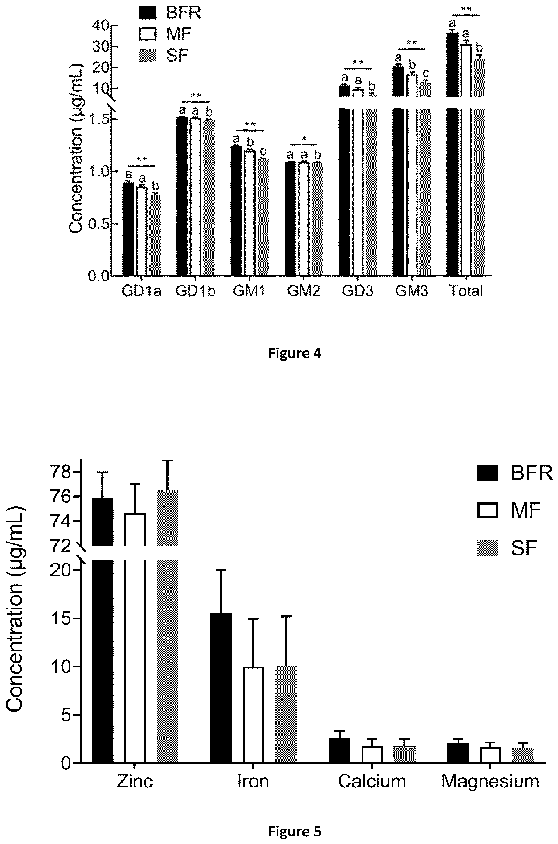

**Figure 4** shows a comparison of the serum ganglioside concentrations (µg/mL;
   presented as mean ± SE) in the MF (white column, n=26), SF (grey column, n=24), and BFR (black column, n=41) groups at 4 months of age. Data were analysed using a univariate general linear model. Group and sex were fixed effects, group by sex interaction and site was used as a random factor. Significant difference is indicated by asterisks *P< 0.05, **P< 0.01. The number at the top of each column shows mean concentration of each ganglioside species.
**Figure 5** shows a comparison of the blood concentrations of zinc, iron, calcium, and magnesium (presented as mean ± SE) in the MF (white column, n=66), SF (grey column, n=60), and BFR (black column, n=122) groups at 12 months of age. Data were analysed using a univariate general linear model. Group and sex were fixed effects, group by sex interaction and site was used as a random factor. MF, MFGM-enriched formula; SF, standard formula; BFR, breast-fed reference. The number at the top each column shows mean concentration of each blood trace elements.

### EXAMPLES

The following non-limiting examples are provided to illustrate the present invention.

### The Study

A prospective, double-blind, four centre, randomised controlled trial was conducted to evaluate the effect of bovine MFGM as a source of gangliosides and phospholipids in the first 12 months of age in healthy term infants in Fuzhou, China. The exclusive breastfeeding rate was about 49-60% across study sites. Neurodevelopmental outcome at 12 months of age was measured using the Bayley-III development test. Growth, neurodevelopmental outcome (Bayley-III) at 6 months of age, attention and short-term memory at 6 and 12 months, and serum ganglioside levels at 4 months, were also evaluated.

The study was conducted in accordance with the guidelines of the Declaration of Helsinki (22), the International Conference on Harmonization Guidelines on Good Clinical Practice, and all other relevant regulations.

### a. Participants

The study population consisted of healthy full-term infants aged < 14 days who were born at the obstetric units and child healthcare units of four hospitals in the Fuzhou region, Fujian Province, China, between January 2016 and October 2016. The inclusion criteria were: (I) healthy newborn male and female infants regardless of mode of delivery (caesarean section or vaginal delivery); (II) gestational age between 37 and 41 weeks; (III) birth weight between 2500 and 4000 g; (IV) intention to predominantly breast feed (breast fed > 90%) or formula feed (formula fed > 60%) during the first 6 months after giving birth; (V) intention to remain in Fuzhou for approximately 15-18 months. The exclusion criteria were: (I) 5-min Apgar score < 7; (II) obvious cerebral and/or other major birth defects or evidence of genetic disease at birth; (III) mothers who were not expected to comply with exclusive breastfeeding or formula feeding. Mothers who were intending to breastfeed or were breastfeeding were invited to take part in the study in the breastfed reference (BFR) group.

The sample size calculation was based on Timby et al.⁽¹⁹⁾, who reported an effect-size of four points (increase) and a standard deviation of nine points on the Bayley-III test between Swedish infants fed MFGM-enriched infant formula and those fed a standard infant formula (n = 80 per group, P= 0.008). Assuming a similar effect size and standard deviation, 88 infants per group were required for a power of 90% and a significance of 5% (based on a two-sided t-test). To allow for 25% dropout, 120 infants per formula-fed group were recruited. To allow for a greater dropout rate, 200 breastfed infants were included.

### b. Randomisation and Blinding

A random permuted block design stratified by site (Fuqing, Fuzhou, and Changle) and by sex was used to generate the allocation schedule. The 2 study formula cans were labelled either in pink or blue. Parents/caregivers, investigators, and the research nurses were aware of the group assignment of breast feeding versus formula feeding. However, they remained blind to the MFGM-enriched formula (MF) until all infants had completed the intervention and analysis of the primary outcome had been conducted.

### c. Formula Composition and Dietary Intake

Infant formulae 0-6 months and follow-on formulae 6-12 months (Fonterra Co-operative Group Limited, New Zealand) were used for the MF and control formula (SF). Supplemented formulae contained minimum ganglioside concentrations of 17.9 mg/100 g (infant formula) and 16.9 mg/100 g (follow-on formula) and were manufactured using a bovine MFGM-rich ingredient as the source of gangliosides and milk phospholipids (SureStart^{™} MFGM Lipid 100; NZMP, Fonterra) from anhydrous milk fat production. The control formula was manufactured with the same macro- and micronutrient composition but without fortification with the MFGM-rich ingredient (Table 1). Both infant formulae met standard nutritional requirements for infants from 0-6 months and 6-12 months(23). At each visit, parents/caregivers were asked to complete a 24-h recall of breastfeeding, formula feeding and intake of complementary foods. We reminded every parent/caregiver to record 24 hours of feeding via phone call or text message 1-2 days before each visit. They were also asked to report any medication or treatment initiated throughout the trial. For product accountability, parents/caregivers in the formula-fed groups were asked to return empty formula cans at each clinical visit.

### d. Assessment of Cognitive Function

The Bayley-III test for global cognitive ability assessment in infants at 6 and 12 months was performed by one trained paediatric psychologist for all participants throughout the trial. The Chinese attention and short-term memory test results from the Bayley-III cognitive assessment were also analysed using a test method validated subsequently and standardised for young Chinese children ^{(24; 25)}(test shown below). These tests were based on 91 subtests of the cognition domain of Bayley III that were separated into "attention (28 subtests)" and "shorten memory (48 subtests)" by a Chinese paediatric specialist in psychiatry and psychology (supplementary data).

### Validated "attention and short-term memory tests" for Chinese children aged 1-42 months. This test method was modified and generated from the Bayley-III cognitive domain subtests

| **Age** | **Number** | **Materials** | **Items¹** | **Psychology** |
|---|---|---|---|---|
| 0.5 mo- | 2 | None | Responds to Surroundings Series: Inspects | **A** |
| 1.5 mo-; 2.5 mo- | 3 | Ring with string | Regards Object for 3 Second | **A** |
| 1.5 mo-; 2.5 mo- | 5 | Bell and stopwatch | Discriminates Between Objects | **A** |
| 3.5 mo- | 7 | None | Becomes Excited in Anticipation | **A** |
| 3.5 mo- | 8 | Block | Regards Object for 5 Seconds | **A** |
| 3.5 mo- | 10 | Bell and rattle | Shifts Attention | **A** |
| 3.5 mo- | 11 | Stimulus book and stopwatch | Shows Visual Preference | **A** |
| 3.5 mo- | 12 | Block | Habituates to Objects | **A** |
| 3.5 mo- | 13 | Block | Prefers Novel Object | **A** |
| 3.5 mo- | 14 | Stimulus book and stopwatch | Habituates to Picture (Balloons) | **A** |
| 3.5 mo- | 15 | Stimulus book and stopwatch | Prefers Novel Picture (Ball) | **A** |
| 4.5 mo- | 16 | Rattle | Explores Object | **A** |
| 4.5 mo- | 18 | None | Inspects Own Hand Responds to Surroundings series: | **A** |
| 5.5 mo- | 20 | None | Awareness of Novelty | **A** |
| 6.5 mo- | 23 | Ring with string | Plays With String | **A** |
| 9.0 mo- | 25 | Squeeze toy | Searches for Fallen Object | **A** |
| 9.0 mo- | 26 | Bell | Bell Series: Manipulates | **A** |
| 9.0 mo- | 29 | Ring with string | Pulls String Adaptively | **A** |
| 11.0 mo- | 31 | Bell | Bell Series: Rings Purposely | **A** |
| 11.0 mo- | 32 | Picture book | Look at Pictures | **A** |
| 16.5 mo- | 41 | Ring with string | Suspends Ring | **A** |
| 16.5 mo- | 44 | Squeeze toy | Squeezes Object | **A** |
| 19.5 mo-; 22.5 mo- | 49 | Pink board | Pink Board Series: 1 Piece | **A** |
| 25.5 mo- | 56 | Pink board | Pink Board Series: Completes | **A** |
| 28.5 mo- | 60 | Pink board | Rotated Pink Board | **A** |
| 0.5 mo- | 1 | None | Calms When Picked up | **M** |
| 1.5 mo-; 2.5 mo- | 4 | Rattle | Habituates to Rattle | **M** |
| 1.5 mo-; 2.5 mo- | 6 | None | Recognizes Caregiver | **M** |
| 3.5 mo- | 9 | None | Reacts to Disappearance of Face | **M** |
| 4.5 mo- | 17 | Glitter bracelet | Carries Object to Mouth | **M** |
| 5.5 mo- | 19 | Mirror | Mirror Image Series: Approaches | **M** |
| 6.5 mo- | 22 | Mirror | Mirror Image Series: Responds Positively | **M** |
| 9.0 mo- | 28 | Washcloth | Pulls Cloth to Obtain Object | **M** |
| 13.5 mo- | 34 | Block | Searches for Missing Objects | **M** |
| 16.5 mo- | 40 | Washcloth | Finds Hidden Object | **M** |
| 19.5 mo-; 22.5 mo- | 45 | Glitter bracelet | Finds Hidden object (Reversed) | **M** |
| 19.5 mo-; 22.5 mo- | 46 | Bottle | Removes Lid From Bottle | **M** |
| 19.5 mo-; 22.5 mo- | 48 | Doll, small ball | Relational Play Series: Self | **M** |
| 19.5 mo-; 22.5 mo- | 50 | Glitter bracelet | Finds Hidden Object (Visible Displacement) | **M** |
| 19.5 mo-; 22.5 mo- | 51 | Blue board | Blue Board Series: 1 Piece | **M** |
| 19.5 mo-; 22.5 mo- | 53 | Doll, small ball | Relational Play Series: Others | **M** |
| 25.5 mo- | 58 | Blue board | Blue Board Series: 4 Pieces | **M** |
| 25.5 mo- | 59 | Book | Attends to story | **M** |
| 33.0 mo- | 64 | Book | Matches Pictures | **M** |
| 33.0 mo- | 65 | Doll, small ball | Representational Play | **M** |
| 33.0 mo- | 66 | Blue board | Blue Board Series: Completes (75 Seconds) | **M** |
| 39.0-42.5 mo | 67 | Imitates | Imitates a Two-Step Action | **M** |
| 39.0-42.5 mo | 68 | Book | Matches 3 Colors | **M** |
| 39.0-42.5 mo | 69 | Doll, small ball | Imaginary Play | **M** |
| 39.0-42.5 mo | 70 | Block | Understands concept of One | **M** |
| 39.0-42.5 mo | 71 | Doll, small ball | Multischeme Combination Play | **M** |
| 39.0-42.5 mo | 72 | Concept colour | Concept Grouping: Color | **M** |
| 39.0-42.5 mo | 73 | Concept size | Concept Grouping: Size | **M** |
| 39.0-42.5 mo | 74 | Compares masses | Compares Masses | **M** |
| 39.0-42.5 mo | 75 | Duck | Matches Size | **M** |
| 39.0-42.5 mo | 76 | Book | Discriminates Pictures | **M** |
| 39.0-42.5 mo | 77 | Duck | Simple Pattern | **M** |
| 39.0-42.5 mo | 79 | Block | Counts (One-to-Once Correspondence) | **M** |
| 39.0-42.5 mo | 80 | Book | Discriminates Sizes | **M** |
| 39.0-42.5 mo | 81 | Book | Identifies 3 Incomplete Pictures | **M** |
| 39.0-42.5 mo | 83 | Book | Discriminates Patterns | **M** |
| 39.0-42.5 mo | 84 | Memory | Spatial Memory | **M** |
| 39.0-42.5 mo | 86 | Block | Number Constancy | **M** |
| 39.0-42.5 mo | 88 | Book | Classifies Objects | **M** |
| 39.0-42.5 mo | 90 | None | Repeats Number Sequences | **M** |

| | | | | |
|---|---|---|---|---|
| ¹Reference Bayley-III cognition domain subsets. | | | | |

In the table above, "Number" refers to the subtest within the Bayley cognition subsets, the exact subtest being age related.

### e. Assessment of Growth

Physical examinations and growth measurements were made at baseline, 42 ± 5 days and 4, 6, 8, and 12 months ± 5 days of age (Figure 1). Weight (HW-B70, Xiamen Zhonghenkang Technology Co. Ltd, China), recumbent length (HW-B70, Xiamen Zhonghenkang Technology Co. Ltd, China), and head circumference were recorded to the nearest 10 g, 0.1 cm, and 0.1 cm respectively at each visit. The WHO Child Growth Standards were used to convert weight, length, and head circumference into weight-for-age, length-for-age, and head-circumference-for-age Z-scores^{(26; 27)}.

### f. Analysis of Serum Gangliosides

One mL of venous blood was randomly collected from 4-month-old infants in the BFR (n= 41), MF (n= 26), and SF groups (n= 24), with written consent from parents/caregivers. Extraction, isolation and identification of serum gangliosides were conducted using our published method⁽²⁸⁾ (Supplementary data). Briefly, the ganglioside species were separated on a Dionex UltiMate 3000 high performance liquid chromatography (HPLC) system (Thermo Scientific, MA, USA) equipped with an APS-2 Hypersil column (150 mm x 2.1 mm, particle size 3 µm; Thermo Electron Corporation, Waltham, MA, USA)⁽²⁸⁾. The sample injection volume was 10 µL. The eluate from the HPLC system was introduced into a Q-Exactive Hybrid Quadrupole-Orbitrap mass spectrometer (Thermo Electron Corporation, Waltham, MA, USA) using a heated electrospray ionization source.

The ganglioside species were confirmed using the HPLC retention time and the precursor ion or the daughter ion of mass spectrum from six ganglioside standards during fragmentation. The list of precursor and daughter ions was created by analyzing chromatograms of ganglioside standards and by referring to the literature⁽²⁹⁾. Total gangliosides were estimated as the sum of the measured major molecular species, i.e. GD1a, GD1b, GD3, GM1, GM2, and GM3. The peak area for each ganglioside species was generated by the Thermo Xcalibur software using accurate mass extract. Quantification of each class of ganglioside was obtained by a specific linear equation generated from external standards. The peaks for GDla (m/z 917 and 931), GDlb (m/z 917 and 931), GD3 (m/z 720 and 775), GM1 (m/z 1544), GM2 (m/z 1382), and GM3 (m/z 1151 and 1235) were used to quantify each class of ganglioside. The molecular species used for the quantification covered all gangliosides found in all serum samples.

### g. Analysis of Blood Trace Elements

The blood iron, zinc, magnesium, and calcium tests are a routine examination for 12 month-old infants in China according to "0∼6 Years Old Children's Health Management Service Standards". These trace elements are essential for the growth and function of the brain(30). Iron deficiency in the early stages of life results not only in acute brain dysfunction, but also in long-lasting abnormalities even after iron repletion(31). Zinc influences the concentration of neurotransmitters in the synaptic cleft, probably via their interaction with neurotransmitter receptors, transporters and ion channels(30). Magnesium plays an essential role in nerve transmission and neuromuscular conduction and protects against neuronal cell death(32). Calcium regulates several neuronal functions, including neurotransmitter synthesis and release, neuronal excitability, and phosphorylation. Calcium is also involved in long-term processes, including memory(33).

Therefore, these blood trace elements were included as co-variates for Bayley III 12 months and group comparisons. Capillary blood (40µL) was collected from the finger of infants. The samples were analyzed within 30 min using an automatic multi-element analyzer (Flame Atomic Absorption Spectrometry; BOHUI, H5300S, China). The concentrations of each trace element were calculated using a standard curve.

### h. Statistical Analysis

Comparison of individual Bayley III outcomes (cognitive, motor, and verbal scores etc), attention and short-term memory scores between MF and SF groups, and secondary pairwise comparisons between 3 groups were analysed using a general linear model with univariate analysis adjusted for socioeconomic factors (maternal age, parental education, and family income), with group and sex as fixed effects, group and sex interaction, and site as a random factor, following the published method (19).

Longitudinal analysis of the main effect of milk intake from 0-12 months, and weight-for-age, recumbent-length-for-age, head-circumference-for -age, and body mass index (BMI)-for-age Z-scores were carried out using general linear mixed model repeated measures analysis of variance (ANOVA) with six time points, adjusted for group, sex as a fixed factors group by sex interaction, and site as a random factor (covariates), which is used to present infant growth from the enrolled date (0) to 12 months of age. To investigate potential different time trends within each group, we included the interaction between time and group in the model. Significant interaction terms indicate that the groups were changing in significantly different ways across time. Comparisons between means (with covariates sex and site) of growth outcome between the groups at six individual time points (14 and 42 days, then 4, 6, 8, and 12 months) and of blood ganglioside analyses were performed with a general linear model ANOVA using Bonferroni's adjustment for post hoc paired comparisons. All analyses were conducted on an intention-to-treat basis, where cases were included in the analysis irrespective of compliance with the intervention.

### RESULTS

### a. Background Characteristics, Compliance, Tolerance, and Dropouts

There were no significant differences in delivery mode, birth weight, birth length, head circumference, and family income at birth between the three groups (Table 2), nor any differences at enrolment between the SF and MF groups, except that the mean maternal weight at pre-delivery of the SF group was 3 and 2 kg heavier than that of the MF and BFR groups respectively (P= 0.045). However, it is important to note that the delivery mode between the MF and the BFR was almost 10% different. BFR mothers were significantly younger, with a high rate of first-time births, and the parents were more educated than those of the formula-fed groups (P ≤ 0.001, Table 2).

Infants (103 girls and 103 boys) were recruited into the BFR group, 108 infants (55 girls and 53 boys) were recruited into the MF group, and 104 infants (55 girls and 49 boys) into the SF group (Figure 1). From initiation, the dropout rates were 14.8% (MF), 20.2% (SF), and 12.1% (BFR). There was no significant difference in dropout rate among the formula-fed groups. The most common reason for discontinuation was related to formula intolerance, as evidenced by constipation (MF, n= 3; SF, n= 5), vomiting (SF, n= 1), and allergic reaction (SF, n= 1). The most common reason for withdrawing from the BFR group was the perception of insufficient milk production. Other reasons were loss of contact, voluntary withdrawal, and inability to follow protocols.

The incidence of gastrointestinal events over 0-12 months was not significantly different across groups (MF, n= 11; SF, n= 14; BFR, n = 12; P=0.055); the percentage of total adverse events were 55% (60/108) in the MF, 47% (49/104) in the SF and 47% (97/208) in the BFR respectively. Over the first 0-6 month period, skin rash (MF, n= 4; SF, n= 0; BFR, n= 12; P= 0.002) and upper respiratory infection (MF, n= 7; SF, n= 1; BFR, n= 15; P= 0.003) were the most frequent adverse events, and constipation (MF, n = 3; SF, n= 2; BFR, n= 0; P= 0.15) and diarrhoea (MF, n= 2; SF, n= 3; BFR, n= 5; P= 0.35) were the most frequent gastrointestinal problems. The incidence of regurgitation and vomiting at 42 days, 4 or 6 months did not vary (P> 0.05) between groups.

### b. Dietary Intake

There were no significant differences in daily intake of formula-milk volume, energy, protein, fat and carbohydrates between the MF and SF groups throughout the study (P= 0.60 - 0.77, Table 3). The MF and SF groups consumed their study formula > 90% of the time between 0-4 months and > 85% between 4-6 months. The milk consumption in the BFR was ∼100% breast milk between 0 - 4 months and > 85% between 4 - 6 months. Formula milk intake in the BFR gradually increased from 15% at 6 months to ∼ 60% at 12 months (Table 3).

### c. Bayley-III Composite and Scaled Scores

At 12 months, the Bayley-III social emotional and adaptive behavior composite scores were 3.50 (95% CI 0.03 to 6.79, P= 0.048) and 5.62 (95% CI 1.78 to 9.38, P= 0.004) points higher in the MF than in the SF group (Table 4). Although the cognitive score was 2.86 points higher in the MF group than in the SF group, the difference was not statistically significant (P= 0.08), even when males and females were analysed separately (Supplementary Table 1A-B). No significant differences in the motor or language domains between the MF and SF groups were found, whether all infants were included in the analysis or when males and females were analysed separately (P= 0.25 - 0.87, Table 4 and Supplementary Tables 1A-B). All composite scores of the BFR group were higher than those for MF and the SF groups at 12 months (P= 0.15 - 1.00, Table 4).

At 12 months of age, 173 BFR infants (95%), 83 MF infants (90%), and 77 SF infants (93%) had composite cognitive scores within the normal range, i.e. ≥ 85. Seven BFR, 7 MF, and 5 SF group infants presented a mild delayed performance with composite cognitive scores between 70 and 85. Two BFR, 2 MF, and 1 SF group infant were classified as expressing severely delayed development (< 70). When the composite scores of the two infants with medically diagnosed severe neurodevelopmental delay (score= 60) were excluded, the social emotional (+3.50) and adaptive behaviour (+5.62) scores were more significantly higher in the MF than in SF at 12 months (P= 0.048 and 0.004 Supplementary Table 2).

There were no differences in all Bayley-III composite scores between the MF and SF groups at 6 months (P= 0.16 - 0.95, Table 5). The fine motor scores for BFR infants were significantly higher than for SF at 6 months (95% CIs 0.36 to 2.21, P= 0.003, Table 5), but not than for MF (P= 0.08, Table 5). Furthermore, when analysed separately, the cognitive (+5.24), motor domain (+7.99), and social emotional (+9.65) scores in male infants (Supplementary Table 3A) and the fine motor (+1.52) score in females (Supplementary Table 3B) of the BFR group were significantly higher than those of SF (P= 0.001 - 0.040), but not MF (P= 0.18 - 0.62, Supplementary Tables 3A-B). The BFR group performed significantly better in expressive language in both male and female, gross motor in males and overall motor domain in females compared with the formula-fed groups (P= 0.001 - 0.041, Supplementary Tables 3A-B).

The composite cognitive (95% CI 3.33 to 6.95; P< 0.001), language (95% CI 3.71 to 5.90; P< 0.001), and motor (95% CI 0.13 to 3.66; P= 0.036) scores increased significantly from 6 to 12 months of age among all three groups, based on a general linear mixed model repeated measures analysis of variance (ANOVA) with two time points adjusted for group and sex as a fixed effect, group and time interaction and site as a random effect (Supplementary Figure 1A-C). Differences in social emotional scores between 6 and 12 months were not significant among the three groups (P= 0.18, Supplementary Figure 1D). The general adaptive scores did not improve from 6 to 12 months of age among the three groups (Supplementary Figure 1E). The results of the pairwise comparisons showed no significant differences in all scores between the MF and SF from 6 to 12 months of age (P= 0.60 - 1.00), but there was a significant difference in the composite cognitive, and in the motor scores between the BFR group and the two formula-fed groups (P= 0.001 - 0.018, data not shown).

### d. Attention and Short-Term Memory

The composite score of attention did not differ between MF and SF (P> 0.05) at both 6 and 12 months of age, although their scores were lower than for BFR at 6 months (P = 0.020), but not at 12 months (P= 0.24, Figure 2). For short-term memory, the mean score for MF (102.15 ± 1.87) was significantly higher than for SF (95.29 ± 1.86) at 12 months (95% CI 1.40 to 12.33, P= 0.002), but not at 6 months (P= 0.35, Figure 2). The same trend was found when the raw scores were analysed separately (Supplementary Figure 2A-B).

### e. Growth Assessment

All investigated growth parameters were within the normal range across groups, both when all infants were included in analyses or when male and female infants were analysed separately (Figure 3A-H). The time trends comparison for the growth parameters in the MF and SF showed that there was not a significant difference in weight-for-age (P= 0.60 and 0.57), length- for-age (P= 0.90 and 0.98), head-circumference-for-age (P= 0.30 and 0.82), and BMI-for-age (P= 0.53 and 0.34) Z-scores in male and female infants. Although the weight-for-age, head-circumference-for-age, and BMI-for-age Z-scores at 42 days in both male and female infants for BFR tended to be higher than those of MF and SF, the overall differences were not significant (P= 0.056 - 0.538, Figure 3A-H). However, the weight-for-age of female infants at 4 months of age was the highest in BFR following by SF and then MF, with the overall difference significant (P= 0.024, Figure 3B). The head-circumference-for-age of BFR male infants was slightly smaller than that of MF (P=0.013, Figure 3E) at 6 months of age and SF at 8 months of age (P=0.025, Figure 3E). The overall difference in the head-circumference-for-age between the three groups in male was statistically significant at 6 months (P=0.016) and 8 months (P=0.027), but this was not observed with females (Figure 3F). At 12 months of age, all anthropometric outcomes were not significantly different among study groups (Figure 3A-H).

### f. Serum Gangliosides

The MF group expressed significantly higher concentrations of serum GM3, GD3, GM1, GD1a, GDlb and the sum of these gangliosides than the SF group (P= 0.001 - 0.045, Figure 4). All ganglioside concentrations were significantly higher in BFR than in SF (P= 0.001 - 0.024, Figure 4). However, no significant differences between the BFR and MF groups (P= 0.06 - 0.47, Figure 4) were observed for the summed and individual serum gangliosides at 4 months, with the exceptions of GM1 and GM3 (P= 0.021 and 0.019, Figure 4).

### g. Blood Trace Elements

MF and SF groups had similar concentrations of blood trace elements at 12 months (P= 0.33-0.79, Figure 5). BFR tended to have higher blood concentrations of iron by 55.6% and 53.6%, calcium 51.6% and 49.2%, and magnesium 28.9% and 31.5% than the MF and SF groups, respectively. However, the overall differences between the groups were not significant (P= 0.45 - 0.81, Figure 5).

### CONCLUSION

Human milk is species-specific and uniquely optimised for the needs of the developing infant. Infant formula based on animal milk cannot mimic the complex nutritional composition of human breast milk, although some infant formulas supplemented with bovine MFGM have already been launched in several markets⁽¹⁵⁾. There is, however, not sufficient evidence for the health benefits of bovine MFGM enriched infant formula on neurodevelopment and growth in the early life of humans. The current study assessed the impact of an MFGM-supplemented infant formula, which supported normal growth, was well tolerated, and gave no indication of adverse effects. The Bayley-III test validated for use in Chinese infants^{(34; 35; 36)} provided the cornerstone for the measurement of cognitive development in this study. The MF group had significantly higher scores for social emotional and general adaptive behaviour at 12 months than the SF group. Although the cognitive score for the MF group was higher than that of the SF group by 2.86 points and 2.96 points either included and unincluded 2 children with severe developmental delays respectively, the difference between MF and SF groups were not significant (P>0.05, Table 4 and Supplementary Table 2). There were no differences between the groups for language and motor skills at 12 months, and no differences between the two formula-fed groups for the composite scores at 6 months. Our findings were slightly different from recent reports by Timby et al.⁽¹⁹⁾ using the same Bayley-III version and Li et al. ⁽²⁰⁾ using an adapted Chinese Bayley-III version, which reported that MFGM supplementation of infant formula improved the mean cognitive scores at 12 months of age by 4.0 (95% CI 1.1 to 7.0) and 8.7 points respectively. Timby et al.⁽¹⁹⁾, however, reported the MFGM formula contained lower energy and lower protein than the control formula. The formula used in the Li et al.⁽²⁰⁾ study was supplemented with both bovine MFGM and lactoferrin. In our study, the two formulae differed only by the addition of the MFGM ingredient. Therefore, the nutrient intakes of infants in the current study were different from those in previously published trials.

In a further study, formula supplemented with a concentrated MFGM complex lipid improved cognitive development using the Griffith Scales at 6 month of age⁽²¹⁾. The present study utilized infant formulae containing similar ganglioside levels as the formula used by Gurnida *et al.*⁽²¹⁾*,* when analysed using the same techniques (Rowan A., personal communication). The two formulae used by Gurnida *et al.* also differed in arachidonic acid content⁽²¹⁾. Thus, neither the nutritional composition of infant formula nor the test method for cognitive function were the same between the current and previous studies. In the current study, other key nutrients for gut and brain development including DHA, prebiotics and probiotics were the same in the two formulae. Their presence may have improved cognitive outcomes in both groups, making it more challenging to observe a large difference due to the supplementation with MFGM.

Our results have shown that BFR infants generally had better performance in the Bayley-III test compared with the two formula-fed groups at 6 and 12 months (Table 4 and 5). At 6 months, the cognitive score of the BFR group was significantly higher than the SF group, but not the MF group (Table 5). MFGM supplemented formula-fed infants were more similar to the BFR group for cognitive scores than the SF group. These findings are consistent with previous studies demonstrating significantly higher mean Bayley-III cognitive scores for breast-fed infants compared to standard formula-fed infants^{(19; 20; 37)}. A recent study using supplements of MFGM together with FOS, inulin, probiotics, and LCPUFAs, did not stimulate neurodevelopment measured as general movement at 2, 3 or 4 months of age, but some differences were reported for visual function at 12 months of age⁽³⁸⁾. In the present study, there was progression in neurodevelopment in the two formula fed groups from 6 to 12 months, which approached the scores for the BFR. To our knowledge, this is the first randomized, controlled, and double-blinded trial to evaluate the effects of supplementation on neurodevelopment in healthy term infants with bovine MFGM alone for 12 months as measured by Bayley-III in Chinese infants.

To further assess the impact of infant feeding on cognitive ability, we analysed the scores of the Chinese attention and short-term memory test generated from the Bayley-III cognitive score (Supplementary Table 4). Our key finding was that short-term memory with MFGM supplementation was 6.86 points higher than for the SF at 12 months (P= 0.008), but not at 6 months (P= 0.94). Importantly MFGM supplementation in infant formula allowed attention and short-term memory to approach the level seen with breast feeding infants.

Gangliosides are important for the growth of neurons, synaptic connections, and memory formation^{(11; 39)}. High concentrations of gangliosides have been detected in both brain⁽⁴⁰⁾ and human breast milk⁽²⁹⁾ at different stages of lactation. The most abundant types of ganglioside in human milk are GM3 and GD3, expressed early and late in lactation, ⁽²⁹⁾. We found the most abundant serum gangliosides in infants at 4 months of age were GM3, followed by GD3, GD1b, GM1, GM2 and GD1a, a finding similar to previous reports^{(21; 41)}. We observed that the BFR group had the highest concentration of the serum gangliosides, followed by the MF group and then the SF group. This suggests that MFGM enrichment of infant formula may increase serum ganglioside concentrations to be more similar to that of the breast-fed infant, potentially supporting improved cognitive development^{(10; 21)}.

Human breast milk is the optimal source of nutrition for the early stage of human life, as it provides all the necessary nutrients for normal growth and development. The finding that all growth outcomes measured in this study at 42 days tended to be higher for BFR than those of MF and SF suggests that human breast milk is a species-specific food and composed of the exact nutrition for a baby's needs at this stage. We have investigated in this study the need for infant formulae to be designed to resemble breast milk as closely as possible in terms of composition and function. The growth results in the current study were within the WHO standards across the three groups. Therefore, the nutrients provided by the MF or SF formulae were sufficient to meet infant needs and were well tolerated.

MFGM-enriched formula in early life supports adequate growth and increases serum gangliosides levels, which may improve social emotional, general adaptive, and short-term memory measures of cognitive development in infancy using the validated Bayley-III assessment. MFGM reduced the gap in cognitive development between breast-fed and formula-fed infants for all sub-scores of the Bayley-III test, suggesting that the MF group might resemble more closely the BFR group. However, the unique properties of human milk cannot be replaced by animal milk based or plant based infant formula for full and optimal development of human infants.

**Table 1. Macronutrient and micronutrient compositions of the infant and follow-on formulae enriched in MFGM (MF) and the control infant and follow-on formulae (SF).**

| **Nutrient per 100 mL** | **MF IF** | **SF IF** | **MF FO** | **SF FO** |
|---|---|---|---|---|
| Energy (kcal) | 67 | 67 | 68 | 68 |
| Proteins (g) | 1.7 | 1.7 | 2.2 | 2.2 |
| Carbohydrate (lactose; g) | 7.4 | 7.4 | 7.8 | 7.8 |
| Fructooligosaccharides (mg) | 58 | 58 | 77 | 77 |
| Lipids (g) | 3.4 | 3.4 | 3.2 | 3.2 |
| Linoleic acid (mg) | 458 | 497 | 408 | 459 |
| α-linolenic acid (mg) | 62 | 53 | N.D. | N.D. |
| Arachidonic acid (mg) | 8.9 | 8.5 | 7.8 | 7.0 |
| DHA (mg)¹ | 8.4 | 8.2 | 11.9 | 10.4 |

| MFGM components: | | | | |
|---|---|---|---|---|
| Phospholipids (mg) | 71.5 | 39.4 | 75.5 | 36.5 |
| Phosphatidylcholine (mg) | 20.6 | 11.0 | 21.3 | 10.3 |
| Phosphatidylethanolamine (mg) | 19.1 | 9.6 | 20.4 | 9.1 |
| Phosphatidylinositol (mg) | 6.9 | 4.5 | 6.9 | 3.9 |
| Phosphatidylserine (mg) | 6.7 | 3.3 | 7.2 | 3.2 |
| Sphingomyelin (mg) | 12.8 | 6.3 | 13.3 | 5.9 |
| Gangliosides (mg)² | 2.5 | 1.4 | 2.7 | 1.5 |
| Probiotic (*Bifidobacterium lactis* HN019; cfu³) | 1.0 x 10⁸ | 1.0 x 10⁸ | 1.0 x 10⁸ | 1.0 x 10⁸ |

| | | | | |
|---|---|---|---|---|
| N.D., not determined; IF, infant formula for 0-6 months; FO, follow-on formula for 6-12 months. ¹Because of batch-to-batch variation and the necessity to produce two batches during the study, 35% of the infants received an MF or SF FO with 7.7 mg DHA/100 mL for the 6-12 months intervention period, and 24, 18, and 23% of the infants received, for the last month, 2 months, and 3 months of the intervention period respectively, an MF or SF FO with 9.6 mg DHA/100 mL. All other nutrients were present at the same levels across batches. ²Measured as GD3 as described in Fong et al. ⁽⁴²⁾. ³cfu: Colony-forming unit. | | | | |

**Table 2. Demographic information for breast-fed reference (BFR), MFGM-enriched formula (MF), and standard formula (SF) groups.**

| | **MF (*n* = 108) M⁴: 53, F⁵: 55** | **SF (*n* = 104) M: 49, F: 55** | **BFR (*n* = 206) M: 103, F: 103** | **P value⁸ Overall** |
|---|---|---|---|---|
| Caesarean section (%) | 45 (41.7%)⁶ | 36 (34.6%) | 67 (32.5%) | 0.17 |
| Birth weight (kg) | 3.21 ± 0.36⁷ | 3.24 ± 0.39 | 3.28 ± 0.33 | 0.18 |
| Birth length (cm) | 49.51 ± 1.72 | 49.63 ± 1.92 | 49.54 ± 1.40 | 0.84 |
| Birth height (cm) | 33.88 ± 1.24 | 33.89 ± 1.38 | 34.15 ± 1.20 | 0.10 |
| Maternal age (y) | 29.38 ± 5.00 | 29.69 ± 5.00 | 27.70 ± 4.56 | 0.001 |
| Maternal weight (kg) | 65.83 ± 8.37 | 68.68 ± 10.35 | 66.73 ± 7.25 | 0.045 |
| Maternal education (%)¹ | | | | < 0.001 |
| Higher education | 25 (23.1%) | 25 (24.0%) | 84 (40.8%) | 0.001 |
| High school | 34 (31.5%) | 27 (26.0%) | 73 (35.4%) | 0.24 |
| Middle school | 41 (38.0%) | 46 (44.2%) | 48 (23.3%) | < 0.001 |
| Primary school | 5 (4.6%) | 5 (4.8%) | 1 (0.5%) | 0.026 |
| Illiterate | 3 (2.8%) | 1 (1.0%) | 0 (0.0%) | 0.06 |
| Paternal education (%) | | | | < 0.001 |
| Higher education | 23 (21.3%) | 25 (24.0%) | 81 (39.3%) | 0.001 |
| High school | 32 (29.6%) | 28 (26.9%) | 70 (34.0%) | 0.42 |
| Middle school | 46 (42.6%) | 44 (42.3%) | 51 (24.8%) | 0.001 |
| Primary school | 7 (6.5%) | 5 (4.8%) | 3 (1.5%) | 0.06 |
| Illiterate | 0 (0.0%) | 2 (1.9%) | 1 (0.5%) | 0.22 |
| Parity (%) | | | | < 0.001 |
| 1 | 34 (31.5%) | 32 (30.8%) | 99 (48.1%) | 0.002 |
| 2 | 59 (54.6%) | 50 (48.1%) | 94 (45.6%) | 0.32 |
| 3 | 11 (10.2%) | 18 (17.3%) | 13 (6.3%) | 0.01 |
| 4+ | 4 (3.7%) | 4 (3.8%) | 0 (0.0%) | 0.019 |
| Family income² (%) | | | | 0.19 |
| >10,000 RMB³ | 17 (15.7%) | 10 (9.6%) | 33 (16.0%) | 0.28 |
| 5,000-10,000 RMB | 47 (43.5%) | 43 (41.3%) | 90 (43.7%) | 0.92 |
| 3,000-5,000 RMB | 38 (35.2%) | 34 (32.7%) | 69 (33.5%) | 0.93 |
| 2,000-3,000 RMB | 3 (2.8%) | 8 (7.7%) | 4 (1.9%) | 0.03 |
| <2,000 RMB | 1 (0.9%) | 2 (1.9%) | 0 (0.0%) | 0.16 |
| Unknown | 2 (1.9%) | 7 (6.7%) | 10 (4.9%) | 0.23 |

| | | | | |
|---|---|---|---|---|
| ¹ Maternal education at delivery. ² Monthly family income. ³RMB: Ren Min Bi the official currency in China. ⁴M: Male ⁵F: Female ⁶ The data in parentheses shows the results in percentage within the group. ⁷Mean ± SE (same for all such values). ⁸A general linear model, group and sex as fixed effects and site as a random factor. | | | | |

**Table 3. Means (± SD) daily intake of formula-milk volume, energy, protein, fat and carbohydrates between MFGM formula and standard formula groups during first year of life¹.**

| Daily intake | | MF | | | SF | | | MF vs SF | |
|---|---|---|---|---|---|---|---|---|---|
| | | No. | Mean | SD | No. | Mean | SD | *P¹* Value | Adjusted *P²* Value |
| FF volume intake (ml/d) | 42d | 96 | 823.17 | 286.91 | 82 | 890.67 | 268.01 | 0.55 | 0.65 |
| | 4M | 92 | 966.74 | 271.09 | 82 | 937.13 | 351.08 | | |
| | 6M | 91 | 959.51 | 294.17 | 81 | 958.46 | 275.55 | | |
| | 8M | 90 | 893.39 | 276.59 | 80 | 908.34 | 276.38 | | |
| Energy (kcal/d) | 12M | 91 | 757.76 | 307.20 | 83 | 829.94 | 280.74 | 0.55 | 0.65 |
| | 42d | 96 | 551.53 | 192.23 | 82 | 596.75 | 179.57 | | |
| | 4M | 92 | 647.72 | 181.62 | 82 | 627.88 | 235.22 | | |
| | 6M | 91 | 642.87 | 197.09 | 81 | 642.17 | 184.62 | | |
| | 8M | 90 | 607.50 | 188.08 | 80 | 617.67 | 187.94 | | |
| | 12M | 91 | 515.29 | 208.89 | 83 | 564.36 | 190.90 | | |
| Protein (g/d) | 42d | 96 | 13.99 | 4.88 | 82 | 15.14 | 4.56 | 0.50 | 0.60 |
| | 4M | 92 | 16.44 | 4.61 | 82 | 15.93 | 5.97 | | |
| | 6M | 91 | 16.31 | 5.00 | 81 | 16.29 | 4.68 | | |
| | 8M | 90 | 19.66 | 6.9 | 80 | 19.98 | 6.08 | | |
| Fat (g/d) | 12M | 91 | 16.67 | 6.76 | 83 | 18.26 | 6.18 | 0.56 | 0.66 |
| | 42d | 96 | 27.99 | 9.76 | 82 | 30.28 | 9.11 | | |
| | 4M | 92 | 32.87 | 9.22 | 82 | 31.86 | 11.94 | | |
| | 6M | 91 | 32.62 | 10.00 | 81 | 32.59 | 9.37 | | |
| | 8M | 90 | 28.59 | 8.85 | 80 | 29.07 | 8.84 | | |
| | 12M | 91 | 24.25 | 9.83 | 83 | 26.56 | 8.98 | | |
| | 42d | 96 | 60.92 | 21.23 | 82 | 65.91 | 19.83 | | |
| Carbohydrate (lactose g/d) | 4M | 92 | 71.54 | 20.06 | 82 | 69.35 | 25.98 | 0.54 | 0.64 |
| | 6M | 91 | 71.00 | 21.77 | 81 | 70.93 | 20.39 | | |
| | 8M | 90 | 69.68 | 21.57 | 80 | 70.85 | 21.56 | | |
| Meal size (ml/time) | 12M | 91 | 59.11 | 23.96 | 83 | 64.74 | 21.90 | 0.64 | 0.70 |
| | 42d | 96 | 97.92 | 19.86 | 82 | 99.39 | 19.99 | | |
| | 4M | 92 | 135.00 | 26.61 | 82 | 132.44 | 31.84 | | |
| | 6M | 91 | 151.98 | 36.33 | 81 | 149.51 | 34.40 | | |
| | 8M | 90 | 162.33 | 39.29 | 80 | 166.19 | 32.21 | | |
| | 12M | 91 | 171.15 | 39.58 | 83 | 180.00 | 36.92 | | |
| Number of daily meals (Time/day) | 42d | 96 | 8.38 | 2.24 | 82 | 8.96 | 2.04 | 0.70 | 0.77 |
| | 4M | 92 | 7.21 | 1.56 | 82 | 7.05 | 1.96 | | |
| | 6M | 91 | 6.37 | 1.50 | 81 | 6.49 | 1.67 | | |
| | 8M | 90 | 5.61 | 1.56 | 80 | 5.46 | 1.49 | | |
| | 12M | 91 | 4.46 | 1.49 | 83 | 4.62 | 1.31 | | |

The main effects between groups were analysed using a general linear mixed model repeated measures analysis of variance (ANOVA) with six time points without adjustment¹ and with adjusted for group and sex as a fixed effect, group and time interaction and site as a random effect².

**Table 4. Bayley-III composite scores (mean ± SE) at 12 months for the MFGM-enriched formula (MF), vs standard formula (SF) and breast-fed reference (BFR) group.**

| | **MF (*n*** = **92)** | | **SF (*n*** = **83)** | | ***P*¹ (adjusted *P*²)** | **BFR (*n*** = **182)** | | ***P*¹ (adjusted *P*²)** | | |
|---|---|---|---|---|---|---|---|---|---|---|
| | | | | | | | | | | |

| | Mean | SE | Mean | SE | **MF vs SF** | Mean | SE | **Overall** | **BFR vs MF** | **BFR vs SF** |
|---|---|---|---|---|---|---|---|---|---|---|
| Cognitive | 97.61 | 1.35 | 94.75 | 1.34 | 0.77 (0.08) | 97.48 | 1.18 | 0.08 (0.14) | - | - |
| Language | 95.20 | 1.00 | 94.81 | 0.99 | 0.27 (0.87) | 96.32 | 0.87 | 0.06 (0.38) | - | - |
| Receptive³ | 9.00 | 0.24 | 8.80 | 0.24 | 0.71 (0.51) | 9.13 | 0.21 | 0.35 (0.49) | - | - |
| Expressive³ | 9.33 | 0.17 | 9.39 | 0.17 | 0.08 (0.61) | 9.57 | 0.15 | **0.012** (0.45) | **0.009** (-) | - |
| Motor | 92.37 | 1.10 | 91.46 | 1.10 | 0.80 (0.49) | 92.29 | 0.97 | 0.06 (0.75) | - | - |
| Fine motor³ | 8.81 | 0.13 | 8.74 | 0.13 | 0.89 (0.58) | 8.91 | 0.12 | 0.43 (0.53) | - | - |
| Gross motor³ | 8.63 | 0.31 | 8.41 | 0.31 | 0.79 (0.56) | 8.50 | 0.28 | 0.08 (0.84) | - | - |
| Social emotional | 94.18 | 1.48 | 90.68 | 1.48 | **0.82 (0.048)** | 93.74 | 1.30 | 0.15 (0.10) | - | - |
| General adaptive | 95.73 | 1.56 | 90.11 | 1.55 | **0.06 (0.004)** | 92.19 | 1.37 | 0.1 **(0.01)** | - | - |

| | | | | | | | | | | |
|---|---|---|---|---|---|---|---|---|---|---|
| ¹*P* value without adjustment. ²*P* value adjusted for maternal age, maternal and paternal education, family income and blood trace elements (iron, zinc, magnesium and calcium) at 12 months (the blood trace elements and Bayley III were measured at same day) using general linear model with univariate analysis group and sex as fixed effects, group and sex interaction, and site as a random factor. A randomized MF and SF comparison and a non-randomized BFR vs EF and SF comparison were analysed separately. ³Subscale with scaled score. | | | | | | | | | | |

**Table 5. Bayley-III composite scores (mean ± SE) at 6 months for the MFGM-enriched formula (MF) vs standard formula (SF) and breast-fed reference (BFR) groups.**

| | **MF (n = 91)** | | **SF (n = 82)** | | ***P*¹ (adjusted *P*²)** | **BFR (n = 182)** | | ***P*¹ (adjusted *P*²)** | | |
|---|---|---|---|---|---|---|---|---|---|---|
| | Mean | SE | Mean | SE | **MF vs SF** | Mean | SE | **Overall** | **BFR vs MF** | **BFR vs SF** |
| Cognitive | 91.64 | 1.17 | 89.68 | 1.23 | 0.26 (0.21) | 93.30 | 0.95 | **0.010** (0.05) | - | **0.009 (-)** |
| Language | 90.53 | 0.60 | 89.31 | 0.63 | 0.18 (0.16) | 90.49 | 0.49 | 0.07 (0.24) | - | - |
| Receptive³ | 7.12 | 0.17 | 6.83 | 0.18 | 0.24 (0.20) | 6.65 | 0.14 | 0.22 (0.08) | - | - |
| Expressive³ | 9.57 | 0.10 | 9.42 | 0.10 | 0.33 (0.37) | 10.06 | 0.08 | **<0.001 (<0.001)** | **<0.001 (<0.001)** | **<0.001 (<0.001)** |
| Motor | 86.24 | 1.45 | 85.08 | 1.53 | 0.56 (0.53) | 92.55 | 1.18 | **<0.001 (<0.001)** | **<0.001 (0.001)** | **<0.001 (<0.001)** |
| Fine motor³ | 7.41 | 0.30 | 6.96 | 0.32 | 0.32 (0.28) | 8.24 | 0.25 | **<0.001 (0.003)** | **0.026 (-)** | **0.001 (0.003)** |
| Gross motor³ | 7.99 | 0.23 | 8.04 | 0.24 | 0.96 (0.95) | 9.24 | 0.19 | **<0.001 (<0.001)** | **<0.001 (<0.001)** | **<0.001 (<0.001)** |
| Social emotional | 90.03 | 1.63 | 89.61 | 1.71 | 0.89 (0.79) | 93.62 | 1.32 | **0.023** (0.08) | - | - |
| General adaptive | 97.12 | 1.10 | 96.93 | 1.15 | 0.97 (0.83) | 98.57 | 0.89 | 0.17 (0.40) | - | - |

| | | | | | | | | | | |
|---|---|---|---|---|---|---|---|---|---|---|
| ¹*P* value without adjustment. ²*P* value adjusted for maternal age, maternal and paternal education, and family income using general linear model with univariate analysis group and sex as fixed effects, group and sex interaction, and site as a random factor. A randomized MF and SF comparison and a non-randomized BFR vs EF and SF comparison were analysed separately. ³Subscale with scaled score. | | | | | | | | | | |

### References

1. Prado EL, Dewey KG (2014) Nutrition and brain development in early life. Nutr Rev 72, 267-284.
2. Bernard JY, De Agostini M, Forhan A et al. Breastfeeding Duration and Cognitive Development at 2 and 3 Years of Age in the EDEN Mother-Child Cohort. 163, 36-42.e31.
3. Victora CG, Bah1 R, Barros AJ et al. (2016) Breastfeeding in the 21st century: epidemiology, mechanisms, and lifelong effect. Lancet 387, 475-490.
4. Anderson JW, Johnstone BM, Remley DT (1999) Breast-feeding and cognitive development: a meta-analysis. Am J Clin Nutr 70, 525-535.
5. Lucas A, Morley R, Cole TJ et al. (1992) Breast milk and subsequent intelligence quotient in children born preterm. Lancet 339, 261-264.
6. Brink LR, Biochemistry BLJTJoN (2018) The role of milk fat globule membranes in behavior and cognitive function using a suckling rat pup supplementation model. **58.**
7. Koletzko B (2016) Human Milk Lipids. Ann Nutr Metab 69 Suppl 2, 28-40.
8. Wei W, Jin Q, Wang X (2019) Human milk fat substitutes: Past achievements and current trends. Prog Lipid Res 74, 69-86.
9. Hanna, Lee, Emily et al. Compositional Dynamics of the Milk Fat Globule and Its Role in Infant Development.
10. Palmano K, Rowan A, Guillermo R et al. (2015) The role of gangliosides in neurodevelopment. Nutrients 7, 3891-3913.
11. Mendez-Otero R, Pimentel-Coelho PM, Ukraintsev S et al. (2013) Role of Gangliosides in Neurological Development and the Influence of Dietary Sources*.*
12. Hussain G, Wang J, Rasul A et al. (2019) Role of cholesterol and sphingolipids in brain development and neurological diseases. Lipids Health Dis 18, 26.
13. Zhang C, Susuki K, Zollinger DR et al. (2013) Membrane domain organization of myelinated axons requires betaII spectrin. J Cell Biol 203, 437-443.
14. Olsen ASB, Faergeman NJ (2017) Sphingolipids: membrane microdomains in brain development, function and neurological diseases. Open Biol 7, 170069.
15. Timby N, Domellof M, Lonnerdal B et al. (2017) Supplementation of Infant Formula with Bovine Milk Fat Globule Membranes. Adv Nutr 8, 351-355.
16. Sara, Moukarzel, Roger et al. Milk Fat Globule Membrane Supplementation in Formula-fed Rat Pups Improves Reflex Development and May Alter Brain Lipid Composition.
17. Vickers MH, Guan J, Gustavsson M et al. (2009) Supplementation with a mixture of complex lipids derived from milk to growing rats results in improvements in parameters related to growth and cognition. Nutr Res 29, 426-435.
18. Jian G, Alastair M, Bertram F et al. Long-Term Supplementation with Beta Serum Concentrate (BSC), a Complex of Milk Lipids, during Post-Natal Brain Development Improves Memory in Rats. 7, 4526-4541.
19. Timby N, Domellof E, Hernell O et al. (2014) Neurodevelopment, nutrition, and growth until 12 mo of age in infants fed a low-energy, low-protein formula supplemented with bovine milk fat globule membranes: a randomized controlled trial. Am J Clin Nutr 99, 860-868.
20. Li F, Wu SS, Berseth CL et al. (2019) Improved Neurodevelopmental Outcomes Associated with Bovine Milk Fat Globule Membrane and Lactoferrin in Infant Formula: A Randomized, Controlled Trial. J Pediatr 215, 24-31 e28.
21. Gurnida DA, Rowan AM, Idjradinata P et al. (2012) Association of complex lipids containing gangliosides with cognitive development of 6-month-old infants. Early Hum Dev 88, 595-601.
22. World Medical A (2013) World Medical Association Declaration of Helsinki: ethical principles for medical research involving human subjects. JAMA 310, 2191-2194.
23. Code ANZFS (2016) Infant formula products. https://wwwlegislationgovau/Details/F2014C01200/Download*.*
24. Feng Y, Zhou H, Zhang Y et al. (2018) Comparison in executive function in Chinese preterm and full-term infants at eight months. Front Med-Prc 12, 164-173.
25. Delcenserie A, Genesee F (2014) Language and memory abilities of internationally adopted children from China: evidence for early age effects. J Child Lang 41, 1195-1223.
26. Group. WMGRS (2006) WHO Child Growth Standards: Length/height-for-age, weight-for-age, weight-for-length, weight-for-height and body mass index-for-age: Methods and development. Geneva: World Health Organization*.*
27. Ebrahim GJJJoTP (2007) WHO child growth standards: head circumference-for-age, arm circumference-for-age, triceps skin fold-for-age and sub scapular skin fold-for-age. 3.
28. Fong B, Norris C, Lowe E et al. (2009) Liquid chromatography-high-resolution mass spectrometry for quantitative analysis of gangliosides. Lipids 44, 867-874.
29. Ma L, MacGibbon AKH, Mohamed HJBJ et al. (2015) Determination of ganglioside concentrations in breast milk and serum from Malaysian mothers using a high performance liquid chromatography-mass spectrometry-multiple reaction monitoring method. International Dairy Journal 49, 62-71.
30. Takeda A (2004) [Essential trace metals and brain function]. Yakugaku Zasshi 124, 577-585.
31. Georgieff MK (2008) The role of iron in neurodevelopment: fetal iron deficiency and the developing hippocampus. Biochem Soc Trans 36, 1267-1271.
32. Kirkland AE, Sarlo GL, Holton KF (2018) The Role of Magnesium in Neurological Disorders. Nutrients 10**.**
33. Drago I, Davis RL (2016) Inhibiting the Mitochondrial Calcium Uniporter during Development Impairs Memory in Adult Drosophila. Cell Rep 16, 2763-2776.
34. Yue A, Jiang Q, Wang B et al. (2019) Concurrent validity of the Ages and Stages Questionnaire and the Bayley Scales of Infant Development III in China. PLoS One 14, e0221675.
35. Luo R, Emmers D, Warrinnier N et al. (2019) Using community health workers to deliver a scalable integrated parenting program in rural China: A cluster-randomized controlled trial. Soc Sci Med 239, 112545.
36. Hua J, Li Y, Ye K et al. (2019) The reliability and validity of Bayley-III cognitive scale in China's male and female children. Early Hum Dev 129, 71-78.
37. Jasani B, Simmer K, Patole SK et al. (2017) Long chain polyunsaturated fatty acid supplementation in infants born at term. Cochrane Database Syst Rev 3, CD000376.
38. Nieto-Ruiz A, Garcia-Santos JA, Bermudez MG et al. (2019) Cortical Visual Evoked Potentials and Growth in Infants Fed with Bioactive Compounds-Enriched Infant Formula: Results from COGNIS Randomized Clinical Trial. Nutrients 11**.**
39. Wang B (2012) Molecular mechanism underlying sialic acid as an essential nutrient for brain development and cognition. Adv Nutr 3, 465S-472S.
40. Wang B, Brand-Miller J, McVeagh P et al. (2001) Concentration and distribution of sialic acid in human milk and infant formulas. Am J Clin Nutr 74, 510-515.
41. Tan S, Zhao A, Fong B et al. (2019) Dietary Intake of Gangliosides and Correlation with Serum Ganglioside Concentration: Cross-Sectional Study among Chinese Toddlers Aged 24-48 Months. 7, 415-426.
42. Fong B, Norris C, McJarrow P (2011) Liquid chromatography-high-resolution electrostatic ion-trap mass spectrometric analysis of GD3 ganglioside in dairy products. International Dairy Journal 21, 42-47.

## Claims

1. Milk fat globule membrane (MFGM) and/or one or more components thereof (together or individually referred to as "products") for improving social-emotional behaviour and/or memory in an infant or child subject.

2. The product of claim 1, wherein the product is administered to a mother during gestation or lactation, or to an infant or child, optionally wherein the product is administered orally.

3. The product of claim 1 or 2, wherein said MFGM and/or one or more components thereof are comprised in a food product or supplement, or wherein the MFGM and/or one or more components thereof constitute an ingredient for further use in the manufacture of food and drink products.

4. The product of any preceding claim, further comprising probiotic(s) and/or prebiotic(s), optionally selected from human milk oligosaccharides, bifidobacteria, lactobacilli, *Bifidobacterium animalis* subsp. *lactis* HN019 (HN019), and *Lactobacillus rhamnosus* HN001 (HN001), and/or further comprising polyunsaturated fatty acids (such as docosahexaenoic acid (DHA), omega 3, omega 6).

5. The product according to any preceding claim, wherein said MFGM and/or one or more components thereof are obtained from or comprised in buttermilk, beta serum, butter serum, high fat milk protein concentrate, high fat milk protein isolate or fractions thereof, and whey products, such as high fat whey protein concentrate, high fat whey protein isolate, optionally wherein any one of buttermilk, beta serum, butter serum, high fat milk protein concentrate, high fat milk protein isolate or fractions thereof, and whey products, such as high fat whey protein concentrate, high fat whey protein isolate constitute sources of enriched-MFGM or constitute MFGM products.

6. The product according to any preceding claim, wherein the improvement in memory is an improvement in short-term memory.

7. The product according to any preceding claim, wherein said improvement in social-emotional behaviour is measured using the Bayley Scales of Infant and Toddler Development, Third Edition (Bayley-III), Chapter 5 "The Bayley-III Social-Emotional Scale", and wherein said improvement in memory is measured using Bayley Scales of Infant and Toddler Development, Third Edition ("Bayley-III"), Chapter 2 "The Bayley-III Cognitive Scale".

8. The product according to any preceding claim, wherein the MFGM lipids comprise one or more phospholipids, one or more sphingolipids, one or more sphingomyelins or derivatives thereof, one or more ceramides, one or more cerebrosides, one or more MFGM proteins, one or more gangliosides, or any combination thereof.

9. The product according to any one of the preceding claims, wherein administration of said product increases serum ganglioside levels in a foetus, infant, or child, optionally wherein levels of any one or more of GM3, GD3, GM1, GDla and GDlb are increased.

10. The product of claim 1, wherein said MFGM components comprise any one or more of GM3, GD3, GM1, GDla and GD1b, optionally in a formulation comprising at least about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9% or at least about 1% gangliosides w/w on a dry weight basis, or at least about 1, 2, 3, 4, 5, 6, 7, 8, 9, 10, 11, 12, 13, 14, 15, 16, 17, 18, 19, 20, 30, 40, 50, 60, 70, 80, 90, 100, 150, 200, 250, 300, 350, 400, 450, 500, 550, 600, 650, 700, 750, 800, 850, 900, 950, 1000, 2000, 3000, 4000, 5000, 6000, 7000, 8000, 9000, or at least about 10000 mg or more gangliosides per 100g dry weight.

11. Use of MFGM and/or one or more of its components in the manufacture of a formulation for improving social-emotional behaviour and/or memory in an infant or child subject, wherein the formulation is administered to a mother during gestation or lactation or to an infant or child subject.

12. Use according to claim 11, wherein the formulation is an infant formula, follow-on formula, a growing up milk, maternal milk or food product, ingredient or supplement.

13. A method for improving social-emotional behaviour and/or memory in an infant or child, comprising administering a composition comprising MFGM and/or one or more of its components to a mother during gestation or lactation, or to an infant or child subject in need thereof.

14. A method of using MFGM and/or one or more of its components for improving social-emotional behaviour and improving memory, the method comprising administering to a pregnant or lactating mother a composition comprising MFGM and/or one or more of its components and optionally informing the mother that the composition will subsequently maintain or improve social-emotional behaviour and/or improve memory in her child in the weeks, months and years following birth.

15. The product according to any one of claims 1 to 10, or use according claim 11 or 12, or method according to claim 13 or 14, wherein the infant or child subject is a child of any age from birth until pre-pubescence, and where the subject is a foetus, the composition is administered to the expectant mother.
